(19) Europäisches Patentamt / European Patent Office / Office européen des brevets

(11) **EP 3 920 188 B1**

(12) **EUROPEAN PATENT SPECIFICATION**

(45) Date of publication and mention
of the grant of the patent:
**11.02.2026 Bulletin 2026/07**

(21) Application number: **21759220.3**

(22) Date of filing: **26.02.2021**

(51) International Patent Classification (IPC):
**G16B 20/30** *(2019.01)*   **G16B 5/20** *(2019.01)*
**G16B 40/30** *(2019.01)*

(52) Cooperative Patent Classification (CPC):
**G16B 15/30; G06N 3/045; G06N 3/0464;
G06N 3/088; G06N 3/09; G06V 10/82;
G06V 20/695;** G16B 40/20

(86) International application number:
**PCT/CN2021/078263**

(87) International publication number:
**WO 2021/203865 (14.10.2021 Gazette 2021/41)**

(54) **MOLECULAR BINDING SITE DETECTION METHOD AND APPARATUS, ELECTRONIC DEVICE AND STORAGE MEDIUM**

VERFAHREN UND VORRICHTUNG ZUR DETEKTION EINER MOLEKÜLBINDUNGSSTELLE, ELEKTRONISCHE VORRICHTUNG UND SPEICHERMEDIUM

PROCÉDÉ ET APPAREIL DE DÉTECTION DE SITE DE LIAISON MOLÉCULAIRE, DISPOSITIF ÉLECTRONIQUE ET SUPPORT DE STOCKAGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.04.2020 CN 202010272124**

(43) Date of publication of application:
**08.12.2021 Bulletin 2021/49**

(73) Proprietor: **Tencent Technology (Shenzhen)
Company Limited
Shenzhen, Guangdong, 518057 (CN)**

(72) Inventors:
• **LI, Xianzhi
Shenzhen, Guangdong 518057 (CN)**
• **CHEN, Guangyong
Shenzhen, Guangdong 518057 (CN)**
• **HENG, Pheng-Ann
Shenzhen, Guangdong 518057 (CN)**
• **ZHANG, Shengyu
Shenzhen, Guangdong 518057 (CN)**

(74) Representative: **Novagraaf International SA
Chemin de l'Echo 3
1213 Onex, Geneva (CH)**

(56) References cited:
CN-A- 110 544 506     CN-A- 110 706 738
CN-A- 111 243 668     US-A1- 2019 385 705

• KRIV�‌K RADOSLAV ET AL: "P2RANK:
Knowledge-Based Ligand Binding Site
Prediction Using Aggregated Local Features", 28
July 2015, ADVANCES IN BIOMETRICS :
INTERNATIONAL CONFERENCE, ICB 2007,
SEOUL, KOREA, AUGUST 27 - 29, 2007 ;
PROCEEDINGS; [LECTURE NOTES IN
COMPUTER SCIENCE; LECT.NOTES
COMPUTER], SPRINGER, BERLIN,
HEIDELBERG, PAGE(S) 41 - 52, ISBN:
978-3-540-74549-5, XP047339491
• STELIOS K MYLONAS ET AL: "DeepSurf: A
surface-based deep learning approach for the
prediction of ligand binding sites on proteins",
ARXIV.ORG, CORNELL UNIVERSITY LIBRARY,
201 OLIN LIBRARY CORNELL UNIVERSITY
ITHACA, NY 14853, 13 February 2020
(2020-02-13), XP081599025

EP 3 920 188 B1

• TIAGO SIM�ES ET AL: "Geometric Detection Algorithms for Cavities on Protein Surfaces in Molecular Graphics: A Survey", COMPUTER GRAPHICS FORUM : JOURNAL OF THE EUROPEAN ASSOCIATION FOR COMPUTER GRAPHICS, WILEY-BLACKWELL, OXFORD, vol. 36, no. 8, 1 June 2017 (2017-06-01), pages 643 - 683, XP071489622, ISSN: 0167-7055, DOI: 10.1111/CGF.13158

• YULAN GUO ET AL: "Deep Learning for 3D Point Clouds: A Survey", ARXIV.ORG, CORNELL UNIVERSITY LIBRARY, 201 OLIN LIBRARY CORNELL UNIVERSITY ITHACA, NY 14853, 27 December 2019 (2019-12-27), XP081566582

**Description**

FIELD OF THE TECHNOLOGY

This disclosure relates to the field of computer technologies, and in particular, to a method and apparatus for detecting a molecule binding site, an electronic device, and a storage medium.

BACKGROUND OF THE DISCLOSURE

With the development of computer technologies, how to detect a binding site of a protein molecule by using a computer is a hot topic in the biomedical field. The binding site of the protein molecule is a location point on the protein molecule at which the protein molecule binds to another molecule, and the binding site of the protein molecule is generally referred to as a protein binding pocket. Determining binding sites of a protein molecule has significance in analyzing a structure and functions of a protein. Therefore, how to accurately detect a binding site in a protein molecule is an important research direction. D1(XP4733949A) discloses P2RANK, a novel machine learning-based method for prediction of ligand binding sites from protein structure. P2RANK uses Random Forests learner to infer ligandability of local chemical neighborhoods near the protein surface which are represented by specific near-surface points and described by aggregating physico-chemical features projected on those points from neighboring protein atoms. The points with high predicted ligandability are clustered and ranked to obtain the resulting list of binding site predictions. D2(XP81599025A) discloses a novel computational method for the prediction of potential binding sites, called DeepDurf. DeepSurf combines a surface-based representation, where a number of 3D voxelized grids are placed on the protein's surface, with state-of-the-art deep learning architectures. After being trained on the large database of scPDB, DeepSurf demonstrates superior performance on two diverse testing datasets, by surpassing all its main deep learning-based competitors.

SUMMARY

Embodiments of this disclosure provide a method and apparatus for detecting a molecule binding site, an electronic device, and a storage medium, to improve the accuracy of a process of detecting a molecule binding site. The technical solutions are as follows:

According to one aspect, a method for detecting a molecule binding site is provided, applicable to an electronic device and including:

obtaining three-dimensional (3D) coordinates of at least one site in a target molecule, the target molecule being a chemical molecule with a to-be-detected binding site;

for each site of the at least one site:

determining a first target point and a second target point, the first target point being a center point of all sites within a spherical space, the spherical space being a spherical space with the respective site as a center of a sphere and a target length as a radius, and the second target point being an intersection between a forward extension line of a vector, starting from an origin and pointing to the respective site, and an outer surface of the spherical space, the origin being an origin of a 3D coordinate system in which the target molecule is located;

extracting a rotation-invariant location feature in the 3D coordinates of the respective site based on the 3D coordinates of the respective site, 3D coordinates of at least one first target point, and 3D coordinates of the second target point, the rotation-invariant location feature being used for indicating location information of the respective site in the target molecule;

invoking a site detection model to perform prediction processing on the extracted rotation-invariant location feature, to obtain a prediction probability of the each site of the at least one site, the each prediction probability indicating a possibility of the each site of the at least one site being a binding site; and

determining a binding site in the at least one site in the target molecule based on prediction probability of the each site of the at least one site.

According to an aspect, an apparatus for detecting a molecule binding site is provided, including:

an obtaining module, configured to obtain 3D coordinates of at least one site in a target molecule, the target molecule being a chemical molecule with a to-be-detected binding site;

for each site of the at least one site:

a first determining module, configured to determine a first target point and a second target point, the first target point being a center point of all sites within a spherical space, the spherical space being a spherical space with the respective site as a center of a sphere and a target length as a radius, and the second target point being an intersection between a forward extension line of a vector, starting from an origin and pointing to the respective site, and an outer surface of the spherical space;

an extraction module, configured to extract a rotation-invariant location feature in the 3D coordinates of the respective site based on the 3D coordinates of the respective site, 3D coordinates of the first target point, and 3D coordinates of the second target point, the rotation-invariant location feature being used for indicating location information of the respective site in the target molecule;

a prediction module, configured to invoke a site detection model to perform prediction processing on the extracted rotation-invariant location feature, to obtain a prediction probability of the each site of the at least one site, the each prediction probability indicating a possibility of the each site of the at least one site being a binding site; and

a second determining module, configured to determine a binding site in the at least one site in the target molecule based on the prediction probability of the each site of the at least one site.

According to an aspect, an electronic device is provided, including one or more processors and one or more memories, the one or more memories storing at least one piece of program code, the at least one piece of program code being loaded and executed by the one or more processors to implement the method for detecting a molecule binding site according to any one of the foregoing possible implementations.

According to an aspect, a non-transitory storage medium is provided, storing at least one piece of program code, the at least one piece of program code being loaded and executed by a processor to implement the method for detecting a molecule binding site according to any one of the foregoing possible implementations.

Beneficial effects brought by the technical solutions provided in the embodiments of this disclosure are at least as follows:

3D coordinates of each site in a target molecule are obtained to determine a first target point and a second target point corresponding to the each site. Based on the 3D coordinates of the each site, 3D coordinates of each first target point, and 3D coordinates of each second target point, a rotation-invariant location feature in the 3D coordinates of the each site is extracted, and a site detection model is invoked to perform prediction on the extracted location feature, to obtain a prediction probability of whether the each site is a binding site, so as to determine a binding site of the target molecule based on the prediction probability. The first target point and the second target point are associated with each site and have spatial representativeness to some extent. Therefore, a rotation-invariant location feature that can completely reflect the detailed structure of the target molecule can be constructed based on the 3D coordinates of the each site, the 3D coordinates of each first target point, and the 3D coordinates of each second target point, thereby avoiding loss of details caused by designing a voxel feature for the target molecule, so that location information of the detailed structure of the target molecule can be fully used during binding site detection based on the location feature, thereby improving the accuracy of a process of detecting a molecule binding site.

BRIEF DESCRIPTION OF THE DRAWINGS

To describe the technical solutions in the embodiments of this disclosure more clearly, the accompanying drawings required for describing the embodiments are briefly described hereinafter. Apparently, the accompanying drawings in the following descriptions show merely some embodiments of this disclosure, and a person of ordinary skill in the art may obtain other accompanying drawings according to these accompanying drawings without creative efforts.

FIG. 1 is a schematic diagram of an exemplary implementation environment of a method for detecting a molecule binding site according to an embodiment of this disclosure.

FIG. 2 is a flowchart of a method for detecting a molecule binding site according to an embodiment of this disclosure.

FIG. 3 is a flowchart of a method for detecting a molecule binding site according to an embodiment of this disclosure.

FIG. 4 is a schematic diagram of a first target point and a second target point according to an embodiment of this disclosure.

FIG. 5 is a schematic principle diagram of a graph convolutional network (GCN) according to an embodiment of this disclosure.

FIG. 6 is a schematic structural diagram of an edge convolutional layer according to an embodiment of this disclosure;

FIG. 7 is a schematic structural diagram of an apparatus for detecting a molecule binding site according to an embodiment of this disclosure.

FIG. 8 is a schematic structural diagram of an electronic device according to an embodiment of this disclosure.

DESCRIPTION OF EMBODIMENTS

To make the objectives, technical solutions, and advantages of this disclosure clearer, implementations of this disclosure are further described below in detail with reference to the accompanying drawings.

Terms such as "first" and "second" in this disclosure are used for distinguishing between same items or similar items that have basically same functions and purposes. It is to be understood that "first", "second", and $n^{th}$ do not have any dependency relationship in logic or in a time sequence, and do not limit a quantity or an execution sequence.

In this disclosure, "at least one" means one or more, and "a plurality of" means two or more. For example, "a plurality of first locations" means two or more first locations.

Artificial intelligence (AI) is a theory, method, technology, and application system that uses a digital computer or a machine controlled by the digital computer to simulate, extend, and expand human intelligence, perceive an environment, obtain knowledge, and use knowledge to obtain an optimal result. In other words, the AI is a comprehensive technology of computer science, which attempts to understand essence of intelligence and produces a new intelligent machine that can respond in a manner similar to human intelligence. The AI is to study the design principles and implementation methods of various intelligent machines, to enable the machines to have the functions of perception, reasoning, and decision-making.

The AI technology is a comprehensive discipline, and relates to a wide range of fields including a hardware-level technology and a software-level technology. The basic AI technologies generally include technologies such as a sensor, a dedicated AI chip, cloud computing, distributed storage, a big data processing technology, an operating/interaction system, and electromechanical integration. AI software technologies mainly include several major directions such as an audio processing technology, a computer vision technology, a natural language processing technology, and machine learning (ML)/deep learning.

The technical solutions provided in the embodiments of this disclosure relate to an ML technology in the AI field. ML is a multi-field interdisciplinary subject involving the probability theory, statistics, the approximation theory, convex analysis, the algorithm complexity theory, and the like. The ML technology specializes in studying how a computer simulates or implements a human learning behavior to obtain new knowledge or skills, and reorganize an existing knowledge structure, so as to keep improving its performance. ML is the core of AI, is a basic way to make the computer intelligent, and is applied to various fields of AI. ML and deep learning generally include technologies such as an artificial neural network, a belief network, reinforcement learning, transfer learning, inductive learning, and learning from demonstrations.

With the research and progress of the ML technology, the ML technology is studied and applied to a plurality of fields. The technical solutions provided in the embodiments of this disclosure relate to the application of the ML technology in the biomedical field, and specifically, to an AI-based method for detecting a molecule binding site. Binding sites are various sites on a current molecule at which the molecule binds to other molecules, and the binding site is generally referred to as a binding pocket or a binding pocket site.

Descriptions are made by using a protein molecule as an example. With the continuous increase in structure knowledge of important protein molecules in biology and medicine, predicting a binding site of a protein molecule becomes an increasingly important hot topic. Molecule functions of proteins may be better revealed by predicting binding sites of the protein molecules. Biological processes are implemented through interaction of protein molecules. Therefore, to fully understand or control a biological process, technicians need to uncover a mechanism behind the protein molecular interaction. For example, a biological process includes deoxyribonucleic acid (DNA) synthesis, signal transduction, life metabolism, and the like. The first step in studying the protein molecular interaction mechanism is to identify an interaction site (that is, a binding site) of the protein molecules. Therefore, predicting the binding site of the protein molecules can assist the technicians in subsequent analysis of the structures and functions of the protein molecules.

Further, predicting the binding site of the protein molecules can help design proper drug molecules. The analysis of the role of the protein molecules greatly helps the progress in the treatment of various diseases. Through the analysis of the structures and functions of the protein molecules, the pathogenesis of some diseases can be revealed, thereby further guiding the search for targets of drugs and research and development of new drugs.

Therefore, predicting the binding site of the protein molecules not only has significance in revealing the structures and functions of the protein molecules, but also can reveal the pathogenesis of some diseases pathologically by revealing the structures and functions of the protein molecules, thereby guiding the search for targets of drugs and research and development of new drugs.

The method for detecting a molecule binding site in the embodiments of this disclosure is used for detecting a binding site of a target molecule. However, the target molecule is not limited to the foregoing protein molecule. The target molecule includes a chemical molecule such as an adenosine triphosphate (ATP) molecule, an organic polymer molecule, or a small organic molecule. The type of the target molecule is not specifically limited in the embodiments of this disclosure.

Terms used in the embodiments of this disclosure are explained in the following.

Protein binding pockets are various binding sites on a protein molecule at which the protein molecule binds to other molecules.

Point cloud data is a data set of points in a specific coordinate system. Data of each point includes rich information, including 3D coordinates, color, intensity, time, and the like of the point. The point cloud data may be obtained by performing data acquisition using a 3D laser scanner.

A deep convolutional neural network (DCNN), as one of representative algorithms of deep learning, is a feedforward neural network that contains convolution calculation and has a deep structure. The structure of the DCNN includes an input layer, a hidden layer, and an output layer. The hidden layer generally includes a convolutional layer, a pooling layer, and a fully-connected layer. The function of the convolutional layer is to perform feature extraction on input data. The convolutional layer includes a plurality of convolution kernels. Each element constituting the convolution kernels corresponds to a weight coefficient and a deviation. After the convolutional layer performs the feature extraction, an outputted feature map is transferred to the pooling layer for feature selection and screening. The fully-connected layer is located at the end of the hidden layer of the DCNN. The feature map loses a spatial topological structure in the fully-connected layer and is unfolded as a vector and transferred to the output layer through an incentive function. An object studied by the DCNN needs to have a regular spatial structure, for example, an image or a voxel.

A graph convolutional network (GCN) is a method for deep learning in graph data. The GCN constructs graph data having points and edges for input data, and extracts a high-dimensional feature for each of the points by using a plurality of hidden layers. The feature implies a graph connection relationship between the point and surrounding points. Finally, an expected output result is obtained by using the output layer. The GCN makes achievements in many tasks such as an e-commence recommendation system, new drug research and development, and point cloud analysis. The GCN network structure includes a spectral convolutional neural network (CNN), a graph attention network, a graph recurrent attention network, a dynamic graph CNN (DGCNN), and the like. A conventional GCN has no rotation invariance.

A multilayer perceptron (MLP) is a forward-structure artificial neural network that can map a group of input vectors to a group of output vectors.

Using a protein molecule as an example, a DCNN is used detecting a protein molecule binding site (protein binding pocket). In recent years, the DCNN performs well in fields such as image and video analysis, recognition, and processing. Therefore, it is attempted to transfer the DCNN to a task of recognizing a protein binding pocket. Although the conventional DCNN made achievements in many tasks, an object studied by the DCNN, such as an image pixel or a molecule voxel, needs to have a regular spatial structure. For much data that does not have a regular spatial structure (for example, a protein molecule) in real life, to transfer the DCNN to a detection process of a protein binding pocket, technicians need to manually design a feature having a regular spatial structure for the protein molecule and use the feature as an input of the DCNN. For example, when the protein binding pocket is detected, a voxel feature needs to be designed for the protein molecule, and then the voxel feature is inputted into the DCNN, to predict, by using the DCNN, whether a molecule structure corresponding to the input voxel feature is a protein binding pocket. Such a process is considered as processing a binary classification problem by using a DCNN.

In an embodiment, a DeepSite network may be the first DCNN put forward for detecting a protein binding pocket. A feature is manually designed (which is essentially a substructure) from a protein molecule as an input of the DCNN, and a multilayer CNN is used for predicting whether the input substructure of the protein molecule is a pocket binding site. Subsequently, in another embodiment, technicians further provide a new feature extraction device that performs feature extraction from two aspects: the shape of the protein molecule and energy of a binding site. An outputted feature is inputted into the DCNN in the form of a 3D voxel (that is, a voxel feature). Similarly, in another embodiment, FRSite is also a DCNN for detecting a protein binding pocket. A voxel feature is extracted from the protein molecule as an input of the DCNN, and a fast CNN is used for binding site detection. Similarly, in another embodiment, deep drop 3D is also a DCNN for detecting a protein binding pocket. The protein molecule is directly converted into a 3D voxel used as an input of the DCNN, to further predict the protein binding pocket.

However, the foregoing DCNN detection methods based on voxel features are severely limited by the resolution of voxels, and thus cannot process a finer protein molecule structure. Furthermore, the voxel features need to be manually designed in the methods as inputs of the DCNN. Although such voxel features are carefully designed by technicians, it still cannot be ensured that important information implied in the protein molecule is fully expressed. Therefore, a final detection result of the protein binding pocket is generally limited by an extraction method for the designed voxel feature.

In view of this, the embodiments of this disclosure provide a method for detecting a molecule binding site for detecting a binding site of a target molecule. Descriptions are made by using an example in which the target molecule is a protein molecule. Point cloud data (including 3D coordinates) of the protein molecule is directly used as a system input, and a site detection model such as a GCN is used for independent exploration. The site detection model can fully explore an organization structure of the protein molecule, so as to automatically extract a biological feature that is efficient and best for binding pocket detection. Therefore, a protein binding pocket can be accurately recognized from the point cloud data of the protein molecule.

Further, a conventional GCN has no rotation invariance, while a protein molecule can rotate in any form in a 3D space. If a deployed network structure has no rotation invariance, pocket detection results of the same protein molecule before and after a rotation may be significantly different, which greatly reduces detection accuracy of the protein binding pocket. Compared with the conventional GCN, in the embodiments of this disclosure, a 3D coordinate point in the point cloud data of the protein molecule is converted into a rotation-invariant feature (i.e., a location feature), such as an angle or a length. The rotation-invariant location feature, in replacement of the rotatable and changeable 3D coordinate point, is used as the system input, so that a network structure of the site detection model is rotation-invariant. That is, the detection result of the protein binding pocket does not change with a direction of the input point cloud data of the protein molecule. This is a critical feature for the detection process of the protein binding pocket. An application scenario of this embodiment of this disclosure is described in detail below.

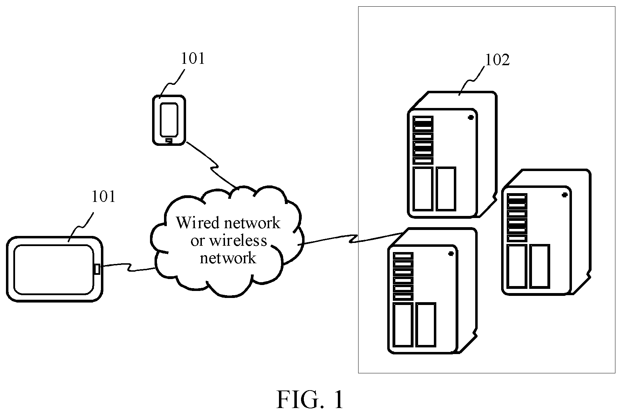

FIG. 1 is a schematic diagram of an implementation environment of a method for detecting a molecule binding site according to an embodiment of this disclosure. Referring to FIG. 1, a terminal 101 and a server 102 are within the implementation environment. Both the terminal 101 and the server 102 are the same electronic device.

The terminal 101 is configured to provide point cloud data of a target molecule. For example, the terminal 101 is a control terminal of a 3D laser scanner. Data acquisition is performed on the target molecule by using the 3D laser scanner, and acquired point cloud data is exported to the control terminal. The terminal is controlled to generate a detection request carrying the point cloud data of the target molecule. The detection request is used for requesting the server 102 to detect a binding site of the target molecule, so that the server 102 detects the binding site for the target molecule based on the point cloud data of the target molecule in response to the detection request, determines the binding site of the target molecule, and returns the binding site of the target molecule to the control terminal.

In the foregoing process, the terminal is controlled to send point cloud data of the entire target molecule to the server 102, so that the server 102 performs a more comprehensive analysis on a molecule structure of the target molecule. In some embodiments, the point cloud data further includes additional attributes such as color, intensity, and time in addition to 3D coordinates of each site. Therefore, in some embodiments, the terminal is controlled to send only 3D coordinates of at least one site of the target molecule to the server 102, thereby reducing a communication volume during a data transmission process.

The terminal 101 and the server 102 may be connected by using a wired network or a wireless network.

The server 102 is configured to provide a detection service of a molecule binding site. After receiving a detection request from any terminal, the server 102 parses the detection request to obtain the point cloud data of the target molecule, extracts a rotation-invariant location feature of each site based on 3D coordinates of the each site in the point cloud data, predicts the binding site by using the location feature as an input of the site detection model, to obtain the binding site of the target molecule.

In some embodiments, the server 102 includes at least one of one server, a plurality of servers, a cloud computing platform, and a virtualization center. In some embodiments, the server 102 is responsible for primary computing, and the terminal 101 is responsible for secondary computing; alternatively, the server 102 is responsible for secondary computing, and the terminal 101 is responsible for primary computing; alternatively, collaborative computing is performed by using a distributed computing architecture between the terminal 101 and the server 102.

In the foregoing process, descriptions are made by using an example in which the terminal 101 interacts with the server 102 through communication to complete the detection of the molecule binding site. In some embodiments, the terminal 101 can also independently complete the detection of the molecule binding site. In this case, after acquiring the point cloud data of the target molecule, based on the 3D coordinates of each site in the point cloud data, the terminal 101 directly preforms prediction based on the site detection model, to predict the binding site of the target molecule. The process is similar to the prediction process of the server 102. Details are not described herein again.

In some embodiments, the terminal 101 is generally one of a plurality of terminals. The device type of the terminal 101 includes but is not limited to at least one of a smartphone, a tablet computer, an ebook reader, a moving picture experts group audio layer III (MP3) player, a moving picture experts group audio layer IV (MP4) player, a portable laptop computer, a desktop computer, or the like. The following embodiment is described by using an example in which the terminal includes a smartphone.

A person skilled in the art learns that, there may be more or fewer terminals 101. For example, there may be only one terminal 101, or there may be more than one terminals 101. The quantity and the device type of the terminals 101 are not limited in the embodiments of this disclosure.

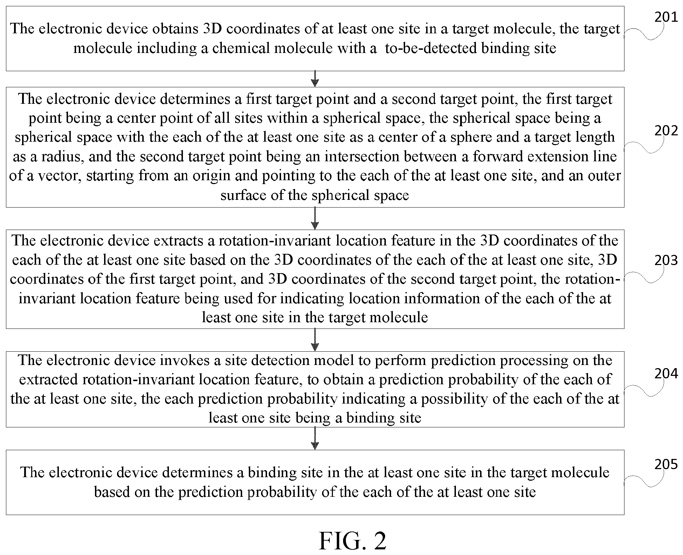

FIG. 2 is a flowchart of a method for detecting a molecule binding site according to an embodiment of this disclosure. Referring to FIG. 2, the method is applicable to an electronic device. The embodiment includes the following steps.

201: The electronic device obtains 3D coordinates of at least one site in a target molecule, the target molecule being a chemical molecule with a to-be-detected binding site.

The target molecule includes any chemical molecule with a binding site to be detected, for example, a protein molecule, an ATP molecule, an organic polymer molecule, or a small organic molecule. The type of the target molecule is not specifically limited in the embodiments of this disclosure.

In some embodiments, the 3D coordinates of the at least one site are represented in the form of point cloud data. The structure of the target molecule is described by stacking at least one 3D coordinate point in a specific coordinate system. Compared with the representation form of a 3D voxel, the point cloud data occupies less storage space. In addition, the 3D voxel depends on a feature extraction manner and thus it is easy to lose some detailed structures in the target molecule during feature extraction. However, the point cloud data can describe the detailed structures of the target molecule.

3D coordinate points are data extremely sensitive to rotations. Using the protein molecule as an example, after a rotation, 3D coordinate values of each site of the same protein point cloud are changed. Therefore, if the 3D coordinates of each site are directly input into a site detection model for feature extraction and binding site prediction, because the coordinate values change before and after the rotation, the same site detection model may extract different biological features from inputs before and after the rotation, and thus predict different binding sites. That is, because the 3D coordinate point has no rotation invariance, the site detection model predicts different binding sites for the same protein molecule before and after the rotation, thus failing to ensure the accuracy of the process of detecting a molecule binding site.

202: The electronic device respectively determines a first target point and a second target point corresponding to each site, the first target point of any site being a center point of all sites within a spherical space, the spherical space being a spherical space with the any site as a center of a sphere and a target length as a radius, and the second target point being an intersection between a forward extension line of a vector, starting from an origin and pointing to the site, and an outer surface of the spherical space.

Each site uniquely corresponds to a first target point and a second target point. For each site, the first target point is a center point of all sites of the target molecule within a target spherical space with the site as a center of a sphere and a target length as a radius. The center point is a space point obtained by calculating an average value of 3D coordinates of all the sites within the target spherical space. Therefore, the first target point is not necessarily a site that actually exists in the point cloud data of the target molecule. The target length may be any value greater than 0. The second target point is an intersection between a forward extension line of a vector, starting from an origin and pointing to the site, and an outer surface of the target spherical space. The origin is an origin of a 3D coordinate system in which the target molecule is located. A vector pointing to the site is derived from the origin. The vector points from the origin to the site. The length of the vector is equal to a magnitude of the site. A forward extension line of the vector has a unique intersection with the outer surface of the target spherical space. The intersection is the second target point. Similarly, the second target point is not necessarily a site that actually exists in the point cloud data of the target molecule.

203: The electronic device extracts a rotation-invariant location feature in the 3D coordinates of the at least one site based on the 3D coordinates of the at least one site, 3D coordinates of the first target point, and 3D coordinates of the second target point, the rotation-invariant location feature being used for indicating location information of the at least one site in the target molecule.

In step 203, the location feature of each site is acquired through the 3D coordinates of the each site, 3D coordinates of each first target point, and 3D coordinates of each second target point. That is, the location feature is not affected by a rotation angle of the target molecule. The location feature replaces the 3D coordinates to be used as the input of the site detection model, thereby avoiding a decrease in the detection accuracy due to the lack of rotation invariance of the 3D coordinates in step 201.

204: The electronic device invokes a site detection model to perform prediction processing on the extracted rotation-invariant location feature, to obtain a prediction probability of the each of the at least one site, the each prediction probability indicating a possibility of the each of the at least one site being a binding site.

The site detection model is used for detecting the binding site of the target molecule. In some embodiments, the site detection model is a classification model, which is used for processing such a classification task as determining whether each site in the target molecule is a binding site. In some embodiments, the site detection model includes a GCN, or includes another deep learning network. The type of the site detection model is not specifically limited in the embodiments of this disclosure.

In step 204, the electronic device inputs the location feature of each site into the site detection model. The site detection model predicts the binding site based on the location feature of each site. In some embodiments, in the site detection model, a biological feature of the target molecule is extracted based on the location feature of each site, and then the binding site is predicted based on the biological feature of the target molecule to obtain a prediction probability of each site.

205: The electronic device determines a binding site in the at least one site in the target molecule based on the prediction probability of the each of the at least one site.

In the foregoing process, the electronic device determines a site with a prediction probability greater than a probability threshold as the binding site, or the electronic device ranks sites according to a descending order of prediction probabilities, and determines a target quantity of top-ranking sites as the binding sites. The probability threshold may be any value greater than or equal to 0 and less than or equal to 1. The target quantity is any integer greater than or equal to 1. For example, when the target quantity is 3, the electronic device ranks the sites according to a descending order of the prediction probabilities. Sites ranked top 3 are determined as the binding sites.

In the method provided in this embodiment of this disclosure, the 3D coordinates of each site in the target molecule are obtained, and the first target point and the second target point corresponding to the each site are determined. Based on the 3D coordinates of the each site, the 3D coordinates of each first target point, and the 3D coordinates of each second target point, the rotation-invariant location feature in the 3D coordinates of the each site is extracted, and the site detection model is invoked to perform prediction on the extracted location feature, to obtain the prediction probability of the each site being a binding site, so as to determine the binding site of the target molecule based on the prediction probability. The first target point and the second target point are associated with each site and have spatial representativeness to some extent. Therefore, a rotation-invariant location feature that can completely reflect the detailed structure of the target molecule can be constructed based on the 3D coordinates of the each site, the 3D coordinates of each first target point, and the 3D coordinates of each second target point, thereby avoiding loss of details caused by designing a voxel feature for the target molecule, so that location information of the detailed structure of the target molecule can be fully used during binding site detection based on the location feature, thereby improving the accuracy of a process of detecting a molecule binding site.

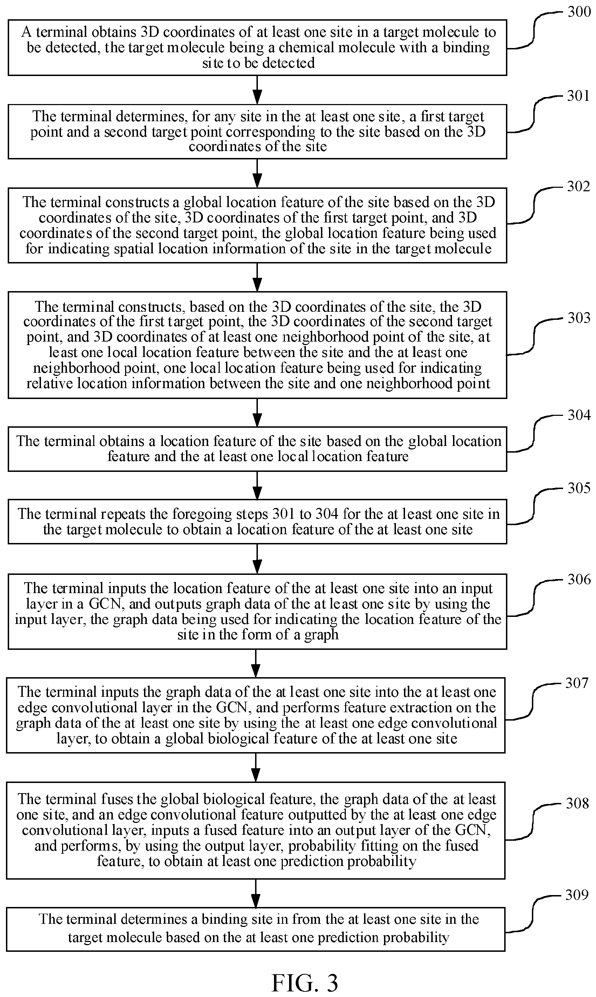

FIG. 3 is a flowchart of a method for detecting a molecule binding site according to an embodiment of this disclosure. Referring to FIG. 3, the method is applicable to an electronic device. Descriptions are made by using an example in which the electronic device is a terminal. The embodiment includes the following steps.

300: The terminal obtains 3D coordinates of at least one site in a target molecule to be detected, the target molecule being a chemical molecule with a binding site to be detected.

Step 300 is similar to step 201, and details are not described herein again.

301: The terminal determines, for any site in the at least one site, a first target point and a second target point corresponding to the site based on 3D coordinates of the site.

Each site corresponds a first target point. For each site, the first target point is a center point of all sites within a target spherical space with the site as a center of a sphere and a target length as a radius. The target spherical space is a spherical space with the site as the center of the sphere and the target length as the radius. The center point is a space point obtained by calculating an average value of 3D coordinates of all the sites within the target spherical space. Therefore, the first target point is not necessarily a site that actually exists in the point cloud data of the target molecule. The target length is specified by technicians and is any value greater than 0.

Each site uniquely corresponds to a second target point. For each site, the second target point is an intersection between a forward extension line of a vector, starting from an origin and pointing to the site, and an outer surface of the target spherical space. A vector pointing to the site is derived from the origin. The vector points from the origin to the site. The length of the vector is equal to a magnitude of the site. A forward extension line of the vector has a unique intersection with the outer surface of the target spherical space. The intersection is the second target point. Similarly, the second target point is not necessarily a site that actually exists in the point cloud data of the target molecule.

In the foregoing process, when determining the first target point and the second target point, the terminal first determines the target spherical space with the site as the center of a sphere and the target length as the radius, then determines all the sites located in the target spherical space from the at least one site in the target molecule, and determines the center point of all the sites located in the target spherical space as the first target point. In some embodiments, when determining the center point, the terminal obtains the 3D coordinates of all the sites located in the target spherical space, determines the average value of the 3D coordinates of all the sites located in the target spherical space as 3D coordinates of the center point, that is, 3D coordinates of the first target point. Further, the terminal determines the vector starting from the origin and pointing to the site, and determines the intersection between the forward extension line of the vector and the outer surface of the target spherical space as the second target point.

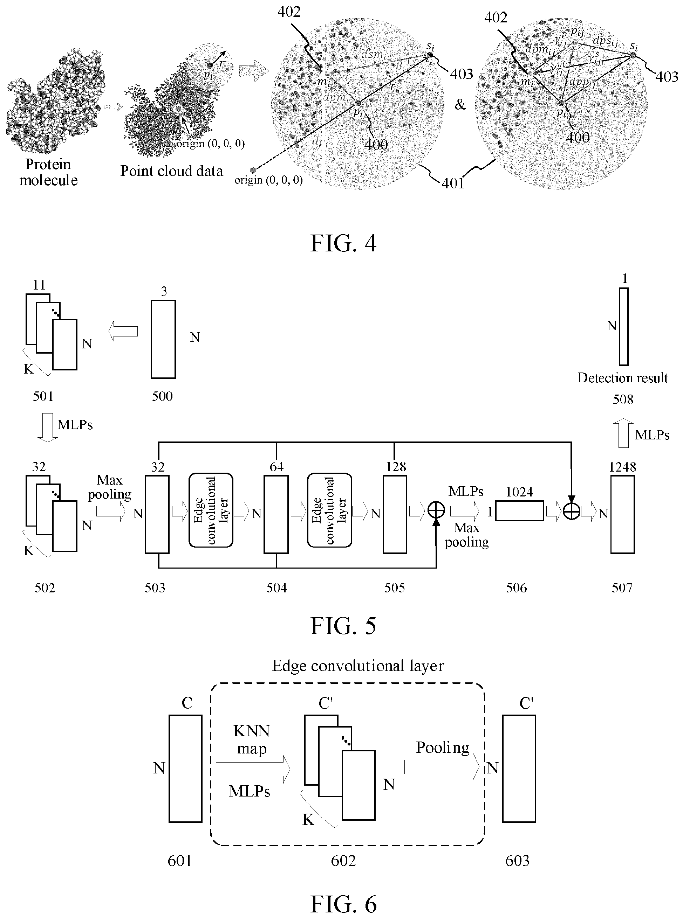

FIG. 4 is a schematic diagram of the first target point and the second target point provided in this embodiment of this disclosure. Referring to FIG. 4, in an embodiment, assuming that point cloud data of a protein molecule includes 3D coordinates of N sites (N is greater than or equal to 1), the point cloud data is obtained by stacking the N 3D coordinates

$$\left\{ p_i = (x_i, y_i, z_i) \right\}_{i=1}^{N}$$ . An origin is (0, 0, 0), $p_i$ represents 3D coordinates of an $i^{th}$ site, $x_i$, $y_i$, and $z_i$ respectively represent the 3D coordinates of the $i^{th}$ site on the x axis, the y axis, and the z axis, and i is an integer greater than or equal to 1 and less than or equal to N. The structure of the protein molecule can be described by using the point cloud data. For the $i^{th}$ site 400, in a target spherical space 401 with $p_i$ as a center of a sphere and r as a radius, a center point $m_i$ of all sites within the target spherical space 401 is determined as a first target point 402. Specifically, an average value of coordinates of all the sites within the target spherical space 401 on the x axis is determined as a coordinate of the center point $m_i$ on the x axis, and an average value of coordinates of all the sites within the target spherical space 401 on the y axis is determined as a coordinate of the center point $m_i$ on the y axis, and an average value of coordinates of all the sites within the target spherical space 401 on the z axis is determined as a coordinate of the center point $m_i$ on the z axis; an intersection $s_i$ between a forward extension line of a vector, starting from the origin and pointing to $p_i$, and an outer surface of the target spherical space 401 is determined as a second target point 403.

302: The terminal constructs a global location feature of the site based on the 3D coordinates of the site, 3D coordinates of the first target point, and 3D coordinates of the second target point, the global location feature being used for indicating spatial location information of the site in the target molecule.

In some embodiments, the global location feature includes at least one of a magnitude of the site, a distance between the site and the first target point, a distance between the first target point and the second target point, a cosine value of a first angle, or a cosine value of a second angle. The first angle is an angle formed between a first line segment and a second line segment, and the second angle is an angle formed between the second line segment and a third line segment. The first line segment is a line segment formed between the site and the first target point, the second line segment is a line segment formed between the first target point and the second target point, and the third line segment is a line segment formed between the site and the second target point.

In some embodiments, the terminal obtains the magnitude of the site, the distance between the site and the first target point, the distance between the first target point and the second target point, the cosine value of the first angle, and the cosine value of the second angle, constructs a 5-dimensional vector based on the five pieces of data, and uses the 5-dimensional vector as the global location feature of the site.

In some embodiments, the global location feature includes at least one of the magnitude of the site, the distance between the site and the first target point, the distance between the first target point and the second target point, a value of the first angle, or a value of the second angle. That is, the operation of obtaining the cosine values of the first angle and the second angle is skipped, and the values of the first angle and the second angle are directly used as elements in the global location feature.

In an embodiment, referring to FIG. 4, for the $i^{th}$ site 400 (which is represented by $p_i$), in the target spherical space 401 using $p_i$ as the center of the sphere and r as the radius, after determining the first target point 402 (which is represented by $m_i$) and the second target point 403 (which is represented by $s_i$) through the foregoing step 301, the terminal respectively obtains the following five pieces of data:

1) a magnitude $dp_i = \|p_i\|^2$ of the site $p_i$;

2) a distance $dpm_i = \| p_i - m_i\|^2$ between the site $p_i$ and the first target point $m_i$;

3) a distance $dsm_i = \|p_i - s_i\|^2$ between the site $p_i$ and the second target point $s_i$ ;

4) a cosine value $\cos(\alpha_i)$ of a first angle $\alpha_i$, where the first angle $\alpha_i$ is an angle formed between a first line segment and a second line segment, the first line segment is a line segment formed between the site $p_i$ and the first target point $m_i$ , and the second line segment is a line segment formed between the first target point $m_i$ and the second target point $s_i$ ; and

5) a cosine value $\cos(\beta_i)$ of a second angle $\beta_i$ , where the second angle $\beta_i$ is an angle formed between the second line segment and a third line segment, and the third line segment is a line segment formed between the site $p_i$ and the second target point $s_i$ .

It can be learned from FIG. 4 that, the first angle $\alpha_i$ and the second angle $\beta_i$ are two interior angles of a triangle $\triangle m_i s_i p_i$. The terminal can construct, based on the five pieces of data 1) to 5), a 5-dimensional vector as a global location feature of the site $p_i$ : $[dp_i; dpm_i; dsm_i; \cos(\alpha_i); \cos(\beta_i)]$.

An analysis is performed based on the foregoing example. For any given site $p_i$ in the point cloud, if a 3D coordinate point $(x_i, y_i, z_i)$ of the site $p_i$ is directly inputted into a site detection model, because the 3D coordinate point has no rotation invariance, the site detection model predicts different results in binding site detection for the same protein molecule, which reduces the accuracy of a binding site detection process.

In some embodiments, assuming that only the magnitude $dp_i = \|p_i\|^2$ of the site $p_i$ is used as a location feature of the site $p_i$, because the magnitude is rotation-invariant, if the magnitude of the site $p_i$ replaces the 3D coordinate point of the site $p_i$ and is inputted into the site detection model, the problem that the 3D coordinate point has no rotation invariance can be resolved. However, actually, the site $p_i$ in cannot be precisely located a space coordinate system of the point cloud by using only the magnitude of the site $p_i$. If only the magnitude is used as the location feature, some location information among sites in the protein molecule is lost.

In some embodiments, assuming that the terminal further extracts four pieces of data $[dpm_i; dsm_i; \alpha_i; \beta_i]$ in addition to the magnitude $dp_i$ of the site $p_i$, obviously, neither the distances $dp_i$, $dpm_i$, and $dsm_i$ nor the angles $\alpha_i$ and $\beta_i$ change with a rotation of the protein molecule, thereby achieving rotation invariance. Based on the foregoing pieces of data, a 5-dimensional vector $[dp_i; dpm_i; dsm_i; \cos(\alpha_i); \cos(\beta_i)]$ is constructed as the global location feature, and the global location feature replaces the 3D coordinate point $(x_i, y_i, z_i)$ to represent the location of the site $p_i$ in the space coordinate system of the point cloud. That is, the site $p_i$ can be precisely located in the space coordinate system of the point cloud based on the global location feature. Therefore, the global location feature can maintain location information of the site $p_i$ to the maximum extent, and the global location feature is rotation-invariant.

Because the point cloud data of the protein molecule is normalized in advance into a target spherical space with the origin as a center of a sphere and 1 as a radius, value ranges of the distances $dp_i$, $dpm_i$, and $dsm_i$ are between 0 and 1, while value ranges of the first angle $\alpha_i$ and the second angle $\beta_i$ are between 0 and $\pi$ ($\alpha_i$ and $\beta_i \in [0, \pi]$). Cosine values of the first angle $\alpha_i$ and the second angle $\beta_i$ are respectively calculated to obtain $\cos(\alpha_i)$ and $\cos(\beta_i)$ with value ranges between 0 and 1, thereby ensuring data input into the site detection model has uniform value ranges, so that the site detection model has more stable training performance and prediction performance.

303: The terminal constructs, based on the 3D coordinates of the site, the 3D coordinates of the first target point, the 3D coordinates of the second target point, and 3D coordinates of at least one neighborhood point of the site, at least one local location feature between the site and the at least one neighborhood point, one local location feature being used for indicating relative location information between the site and one neighborhood point.

In some embodiments, the neighborhood points of the site include K points most adjacent to the site in the target molecule, K being greater than or equal to 1. Alternatively, the neighborhood points of the site are all sites within a target neighborhood of the site. For example, the target neighborhood is a spherical neighborhood, a columnar neighborhood, or the like with the site as a center point. The choice of the neighborhood is not limited in the embodiments of this disclosure.

In some embodiments, for any neighborhood point in the at least one neighborhood point of the site, the local location feature between the site and the neighborhood point includes at least one of a distance between the neighborhood point and the site, a distance between the neighborhood point and the first target point, a distance between the neighborhood point and the second target point, a cosine value of a third angle, a cosine value of a fourth angle, or a cosine value of a fifth angle. The third angle is an angle formed between a fourth line segment and a fifth line segment, the fourth angle is an angle formed between the fifth line segment and a sixth line segment, and the fifth angle is an angle formed between the sixth line segment and the fourth line segment. The fourth line segment is a line segment formed between the neighborhood point and the site, the fifth line segment is a line segment formed between the neighborhood point and the first target point, and the sixth line segment is a line segment formed between the neighborhood point and the second target point.

In some embodiments, for any neighborhood point in the at least one neighborhood point of the site, the terminal obtains the distance between the neighborhood point and the site, the distance between the neighborhood point and the first target point, the distance between the neighborhood point and the second target point, the cosine value of the third angle, the cosine value of the fourth angle, and the cosine value of the fifth angle, constructs a 6-dimensional vector based on the six pieces of data, and uses the 6-dimensional vector as a local location feature of the site. Further, similar operations are performed for all neighborhood points to obtain local location features of the site relative to all the neighborhood points.

In some embodiments, for any neighborhood point in the at least one neighborhood point of the site, the local location feature between the site and the neighborhood point includes at least one of the distance between the neighborhood point and the site, the distance between the neighborhood point and the first target point, the distance between the neighborhood point and the second target point, a value of the third angle, a value of the fourth angle, or a value of the fifth angle. That is, the operation of obtaining the cosine values of the third angle, the fourth angle, and the fifth angle is skipped, and the values of the third angle, the fourth angle, and the fifth angle are directly used as elements in the local location feature.

In an embodiment, referring to FIG. 4, for the i$^{th}$ site 400 (which is represented by $p_i$), in the target spherical space 401 using $p_i$ as the center of the sphere and r as the radius, the first target point 402 (which is represented by $m_i$) and the second target point 403 (which is represented by $s_i$) can be determined through the foregoing step 301. Assuming that there is a j$^{th}$ (j is greater than or equal to 1) neighborhood point $p_{ij}$ of the i$^{th}$ site $p_i$, it can be seen that a tetrahedron can be constructed by using the site $p_i$, the first target point $m_i$, the second target point $s_i$, and the neighborhood point $p_{ij}$. Side lengths of the tetrahedron include a distance $dpp_{ij}$ between the neighborhood point $p_{ij}$ and the site $p_i$ (the length of the fourth line segment), a distance $dpm_{ij}$ between the neighborhood point $p_{ij}$ and the first target point $m_i$ (the length of the fifth line segment), and a distance $dps_{ij}$ between the neighborhood point $p_{ij}$ and the second target point $s_i$ (the length of the sixth line segment). Angles of the tetrahedron include a third angle $\gamma_{ij}^m$, a fourth angle $\gamma_{ij}^p$, and a fifth angle $\gamma_{ij}^s$. The third angle $\gamma_{ij}^m$ is an angle formed between the fourth line segment $dpp_{ij}$ and the fifth line segment $dpm_{ij}$, the fourth angle $\gamma_{ij}^p$ is an angle formed between the fifth line segment $dpm_{ij}$ and the sixth line segment $dps_{ij}$, and the fifth angle $\gamma_{ij}^s$ is an angle formed between the sixth line segment $dps_{ij}$ and the fourth line segment $dpp_{ij}$.

Further, cosine values of the third angle $\gamma_{ij}^m$, the fourth angle $\gamma_{ij}^p$, and the fifth angle $\gamma_{ij}^s$ are respectively calculated to obtain cosine values $\cos\left(\gamma_{ij}^m\right)$, $\cos\left(\gamma_{ij}^p\right)$, and $\cos\left(\gamma_{ij}^s\right)$ corresponding to the three angles. The 6-dimensional vector $\left[dpm_{ij}; dpp_{ij}; dps_{ij}; \cos\left(\gamma_{ij}^p\right); \cos\left(\gamma_{ij}^m\right); \cos\left(\gamma_{ij}^s\right)\right]$ is constructed as the local location feature between the site $p_i$ and the neighborhood point $p_{ij}$. The local location feature can describe a relative location relationship between the site $p_i$ and the neighborhood point $p_{ij}$ in the space coordinate system of the point cloud. The location information of the site $p_i$ in the space coordinate system of the point cloud of the protein molecule can be described more comprehensive and more precisely by using the global location feature and the local location feature.

304: The terminal obtains a location feature of the site based on the global location feature and the at least one local location feature.

In the foregoing step 302, the terminal obtains a 5-dimensional global location feature. In the foregoing step 303, the terminal obtains at least one 6-dimensional local location feature. For each local location feature, the local location feature is concatenated to the global location feature, to obtain an 11-dimensional location feature component. A matrix constructed by all location feature components is determined as the location feature of the site.

In the foregoing steps 302 to 304, for each site in the target molecule, the terminal can extract a location feature of the site based on 3D coordinates of the site, 3D coordinates of the first target point, and 3D coordinates of the second target point. In this embodiment of this disclosure, descriptions are only made by using an example in which the location feature includes the global location feature and the local location feature. In some embodiments, the location feature is equivalent to the global location feature. That is, after the terminal performs the operation of obtaining the global location feature in step 302, the foregoing steps 303 and 304 are skipped, and global location features of all the sites are directly inputted into the site detection model without obtaining local location features of all the sites, thereby simplifying the process of the binding site detection method and reducing a calculation amount in the binding site detection process.

In an example, for the i$^{th}$ site $p_i$ of the target molecule, there are the first target point $m_i$, the second target point $s_i$, and K (K is greater than or equal to 1) neighborhood points $\left\{p_{ij}\right\}_{j=1}^{K}$ corresponding to the site $p_i$. A 5-dimensional (5-dim) global location feature $[dp_i; dpm_i; dsm_i; \cos(\alpha_i); \cos(\beta_i)]$ is extracted through the foregoing step 302, and K 6-dimensional (6-dim) local location features $\left[dpm_{ij}; dpp_{ij}; dps_{ij}; \cos\left(\gamma_{ij}^p\right); \cos\left(\gamma_{ij}^m\right); \cos\left(\gamma_{ij}^s\right)\right]$ respectively corresponding to the K neighborhood points are extracted through the foregoing step 303. The local location features are concatenated to the global location feature to obtain K 11-dimensional location feature components, to construct a [K*11]-dimensional rotation-invariant location feature. The location feature is expressed as follows:

$$
K\begin{bmatrix}
\overbrace{G_i = \{dp_i, dpm_i, ..., \cos(\beta_i)\}}^{5-dim} & \overbrace{L_{i1} = \{dpm_{i1}, dpp_{i1}, ..., \cos(\gamma_{i1}^s)\}}^{6-dim} \\
G_i = \{dp_i, dpm_i, ..., \cos(\beta_i)\} & L_{i2} = \{dpm_{i2}, dpp_{i2}, ..., \cos(\gamma_{i2}^s)\} \\
... & \\
G_i = \{dp_i, dpm_i, ..., \cos(\beta_i)\} & L_{iK} = \{dpm_{iK}, dpp_{iK}, ..., \cos(\gamma_{iK}^s)\}
\end{bmatrix}
$$

It can be learned from the location feature in the form of a matrix that, the left side of the matrix indicates a global location feature $G_i$ of the site $p_i$, to indicate the location of the site $p_i$ in the point cloud space. The right side of the matrix indicates the K local location features $L_{i1}$ to $L_{iK}$ between the site $p_i$ and the K neighborhood points $p_{i1}$ to $p_{iK}$ of the site, to indicate relative locations between the site $p_i$ and the K neighborhood points $p_{i1}$ to $p_{iK}$ of the site.

305: The terminal repeats the foregoing steps 301 to 304 for the at least one site in the target molecule to obtain a location feature of the at least one site.

In the foregoing steps 301 to 305, the terminal can extract a rotation-invariant location feature in the 3D coordinates of the at least one site based on the 3D coordinates of the at least one site, 3D coordinates of at least one first target point, and 3D coordinates of at least one second target point, the location feature being used for indicating location information of the at least one site in the target molecule. In other words, the terminal constructs, by using 3D coordinates of each site, a location feature that can fully indicate location information of the each site and is rotation-invariant, to achieve a relatively high feature expression capability.

306: The terminal inputs the location feature of the at least one site into an input layer in a GCN, and outputs graph data of the at least one site by using the input layer, the graph data being used for indicating the location feature of the site in the form of a graph.

In this embodiment of this disclosure, descriptions are made by using an example in which the site detection model is a GCN. The GCN includes an input layer, at least one edge convolutional (EdgeConv) layer, and an output layer. The output layer is used for extracting graph data of each site, the at least one edge convolutional layer is used for extracting a global biological feature of the each site, and the input layer is used for feature fusion and probability prediction.

In some embodiments, the input layer of the GCN includes an MLP and a pooling layer. The terminal inputs the location feature of the at least one site into the MLP in the input layer, and maps the location feature of the at least one site by using the MLP, to obtain a first feature of the at least one site, a dimension quantity of the first feature being greater than a dimension quantity of the location feature; and inputs the first feature of the at least one site into the pooling layer in the input layer, and performs dimension reduction on the first feature of the at least one site by using the pooling layer, to obtain the graph data of the at least one site.

In some embodiments, the pooling layer is a max pooling layer. A maximum pooling operation is performed on the first feature in the max pooling layer. Alternatively, the pooling layer is an average pooling layer, and an average pooling operation is performed on the first feature in the average pooling layer. The type of the pooling layer is not specifically limited in the embodiments of this disclosure.

In the foregoing process, the MLP maps the input location feature to the output first feature, which is equivalent to increasing dimensions of the location feature and extracting the high-dimensional first feature. Dimension reduction is performed on the first feature by using the pooling layer, which is equivalent to performing screening and selection on the first feature, where some unimportant information is removed to obtain the graph data.

FIG. 5 is a schematic principle diagram of the GCN provided in this embodiment of this disclosure. Referring to FIG. 5, assuming that [N*3]-dimensional point cloud data 500 of the protein molecule is given, the point cloud data is converted into an [N*K*11]-dimensional rotation-invariant feature 501 by using a rotation-invariance feature extraction device (which is similar to step 301). The rotation-invariant feature 501 is a location feature of each site. Next, a [N*K*32]-dimensional first feature 502 is further extracted based on the originally inputted [N*K*11]-dimensional rotation-invariant feature 501 by using the MLP, and max pooling is performed on the [N*K*32]-dimensional first feature 502 along a direction of K dimensions by using the max pooling layer, to convert the [N*K*32]-dimensional first feature 502 into [N*32]-dimensional graph data 503.

307: The terminal inputs the graph data of the at least one site into the at least one edge convolutional layer in the GCN, and performs feature extraction on the graph data of the at least one site by using the at least one edge convolutional layer, to obtain a global biological feature of the at least one site.

In some embodiments, in the process of extracting the global biological feature, the terminal performs the following sub-steps 3071 to 3074.

3071: The terminal performs, for any edge convolutional layer in the at least one edge convolutional layer, feature extraction by using the edge convolutional layer, on an edge convolutional feature outputted by a previous edge convolutional layer, and inputs an extracted edge convolutional feature into a next edge convolutional layer.

In some embodiments, each edge convolutional layer includes an MLP and a pooling layer. A cluster map is constructed for the any edge convolutional layer based on the edge convolutional feature outputted by the previous edge convolutional layer. The cluster map is inputted into an MLP in the edge convolutional layer, and is mapped by using the MLP, to obtain an intermediate feature of the cluster map. The intermediate feature is inputted into a pooling layer in the edge convolutional layer, and then dimension reduction is performed on the intermediate feature by using the pooling layer. The dimension-reduced intermediate feature is inputted into the next edge convolutional layer.

In some embodiments, in a process of constructing the cluster map, the cluster map is constructed by using a k-nearest neighbor (KNN) algorithm for the edge convolutional feature outputted by the previous convolutional layer. In this case, the constructed cluster map is referred to as a KNN map. Certainly, the cluster map can be constructed by using a k-means algorithm. The method of constructing the cluster map is not specifically limited in the embodiments of this disclosure.

In some embodiments, the pooling layer is a max pooling layer. A maximum pooling operation is performed on the intermediate feature in the max pooling layer. Alternatively, the pooling layer is an average pooling layer, and an average pooling operation is performed on the intermediate feature in the average pooling layer. The type of the pooling layer is not specifically limited in the embodiments of this disclosure.

FIG. 6 is a schematic structural diagram of the edge convolutional layer provided in this embodiment of this disclosure. Referring to FIG. 6, in any edge convolutional layer, for any [N*C]-dimensional edge convolutional feature 601 outputted by a previous convolutional layer, a cluster map (KNN map) is constructed by using a KNN algorithm. A high-dimensional feature is extracted from the cluster map by using an MLP, so that the [N*C]-dimensional edge convolutional feature 601 can be mapped into an [N*K*C']-dimensional intermediate feature 602. Dimension reduction is performed on the [N*K*C']-dimensional intermediate feature 602 by using a pooling layer, to obtain an (N*C']-dimensional edge convolutional feature 603 (dimension-reduced intermediate feature). The [N*C']-dimensional edge convolutional feature 603 is inputted into a next edge convolutional layer.

In the foregoing process, the terminal performs the foregoing operation for each edge convolutional layer in the at least one edge convolutional layer. An edge convolutional feature outputted by a previous edge convolutional layer is used as an input of a next edge convolutional layer. In this way, by using the at least one edge convolutional layer, a series of higher-dimensional feature extraction is performed on the graph data of the at least one site.

In an example, referring to FIG. 5, in an example in which the GCN includes two edge convolutional layers, the terminal inputs [N*32]-dimensional graph data 503 into the first edge convolutional layer, and outputs an [N*64]-dimensional edge convolutional feature 504 by using the first edge convolutional layer. The terminal inputs the [N*64]-dimensional edge convolutional feature 504 into the second edge convolutional layer, outputs an [N*128]-dimensional edge convolutional feature 505 by using the second edge convolutional layer, and performs the following step 3072.

3072: The terminal concatenates the graph data of the at least one site and at least one edge convolutional feature outputted by the at least one edge convolutional layer, to obtain a second feature.

In the foregoing process, the terminal concatenates graph data of each site and an edge convolutional feature outputted by each edge convolutional layer, to obtain the second feature. The second feature is equivalent to a residual feature of the at least one edge convolutional layer, so that not only an edge convolutional feature outputted by the last edge convolutional layer is considered, but also the originally inputted graph data of each site and the edge convolutional feature outputted by each intermediate edge convolutional layer can be considered during the extraction of the global biological feature, thereby helping improve an expression capability of the global biological feature.

The concatenation herein is to dimensionally connect the graph data to the edge convolutional feature outputted by each edge convolutional layer. For example, assuming that there is one edge convolutional layer, [N*32]-dimensional graph data is concatenated to an [N*64]-dimensional edge convolutional feature, to obtain an [N*96]-dimensional second feature.

In an example, referring to FIG. 5, in an example in which the GCN includes two edge convolutional layers, the terminal concatenates the [N*32]-dimensional graph data 503, the [N*64]-dimensional edge convolutional feature 504 outputted by the first edge convolutional layer, and the [N*128]-dimensional edge convolutional feature 505 outputted by the second edge convolutional layer, to obtain an [N*224]-dimensional second feature.

3073: The terminal inputs the second feature into an MLP, and maps the second feature by using the MLP, to obtain a third feature.

In the foregoing process, a process in which the terminal performs feature mapping by using the MLP is similar to the processes of performing feature mapping by using MLPs in the foregoing steps. Details are not described herein again.

3074: The terminal inputs the third feature into a pooling layer, and performs dimension reduction on the third feature by using the pooling layer, to obtain the global biological feature.

In some embodiments, the pooling layer is a max pooling layer. A maximum pooling operation is performed on the third feature in the max pooling layer. Alternatively, the pooling layer is an average pooling layer, and an average pooling operation is performed on the third feature in the average pooling layer. The type of the pooling layer is not specifically limited in the embodiments of this disclosure.

In an example, referring to FIG. 5, the [N*224]-dimensional second feature is inputted into the MLP and the max pooling layer in sequence, to obtain a [1*1024]-dimensional global biological feature 506 of a protein point cloud. Step 308 is performed.

308: The terminal fuses the global biological feature, the graph data of the at least one site, and an edge convolutional feature outputted by the at least one edge convolutional layer, inputs a fused feature into the output layer of the GCN, and performs, by using the output layer, probability fitting on the fused feature, to obtain at least one prediction probability.

Each prediction probability is used for indicating a possibility of a site being a binding site.

In some embodiments, in a process of performing probability fitting on the fused feature, the fused feature is inputted into an MLP in the output layer and is mapped by using the MLP, to obtain the at least one prediction probability. A mapping process using the MLP is similar to the mapping processes using MLPs in the foregoing steps. Details are not described herein again.

In the foregoing process, the terminal fuses the global biological feature, the graph data of each site, and the edge convolutional feature outputted by each edge convolutional layer, and finally performs probability fitting on the fused feature by using the MLP, to fit a prediction probability of the each site being a binding site. In some embodiments, the fusing process is to directly concatenate the global biological feature, the graph data of each site, and the edge convolutional feature outputted by each edge convolutional layer.

In an embodiment, referring to FIG. 5, in an example in which the GCN includes two edge convolutional layers, the terminal concatenates the [N*32]-dimensional graph data 503, the [N*64]-dimensional edge convolutional feature 504 outputted by the first edge convolutional layer, the [N* 128]-dimensional edge convolutional feature 505 outputted by the second edge convolutional layer, and the [1*1024]-dimensional global biological feature 506, to obtain a [1*1248]-dimensional fused feature 507, inputs the [1*1248]-dimensional fused feature 507 into the MLP, and fits, for each site by using the MLP, a prediction probability of the site being a binding site. A finally outputted detection result is an [N*1]-dimensional array 508. Each value in the array 508 represents a prediction probability of a site being a binding site. In the foregoing process, because it needs to be predicted whether each site in the input protein molecule is a binding site, the task is considered as a point-by-point division task.

In the foregoing steps 306 to 308, by using an example in which the site detection model is a GCN, a process of invoking, by the terminal, the site detection model to perform prediction processing on the extracted location feature, to obtain at least one prediction probability of the at least one site is shown. In some embodiments, the site detection model is another deep learning network. The type pf the site detection model is not specifically limited in the embodiments of this disclosure.

309: The terminal determines a binding site from the at least one site in the target molecule based on the at least one prediction probability.

In the foregoing process, the terminal determines a site with a prediction probability greater than a probability threshold from the at least one site as the binding site, or the terminal ranks sites according to a descending order of prediction probabilities, and determines a target quantity of top-ranking sites as the binding sites.

The probability threshold is any value greater than or equal to 0 and less than or equal to 1. The target quantity is any integer greater than or equal to 1. For example, when the target quantity is 3, the electronic device ranks the sites according to a descending order of the prediction probabilities. Sites ranked top 3 are determined as the binding sites.

In the method provided in this embodiment of this disclosure, the 3D coordinates of each site in the target molecule are obtained, and the first target point and the second target point corresponding to the each site are determined. Based on the 3D coordinates of the each site, the 3D coordinates of each first target point, and the 3D coordinates of each second target point, the rotation-invariant location feature in the 3D coordinates of the each site is extracted, and the site detection model is invoked to perform prediction on the extracted location feature, to obtain the prediction probability of the each site being a binding site, so as to determine the binding site of the target molecule based on the prediction probability. The first target point and the second target point are associated with each site and have spatial representativeness to some extent. Therefore, a rotation-invariant location feature that can completely reflect the detailed structure of the target molecule can be constructed based on the 3D coordinates of the each site, the 3D coordinates of each first target point, and the 3D coordinates of each second target point, thereby avoiding loss of details caused by designing a voxel feature for the target molecule, so that location information of the detailed structure of the target molecule can be fully used during binding site detection based on the location feature, thereby improving the accuracy of a process of detecting a molecule binding site.

In this embodiment of this disclosure, the biological feature of the protein molecule is extracted by using powerful performance of the GCN in deep learning, instead of artificially designing a voxel feature as a biological feature by a technician, thereby obtaining a biological feature having a stronger expression capability, and achieving higher accuracy of binding site recognition. In addition, the prediction of a binding site can be completed by using a graphics processing unit (GPU), which can meet a requirement of real-time detection. Further, because a location feature of each site is rotation-invariant, even if the protein molecule rotates, a stable prediction result can still be generated by using the GCN, thereby improving the accuracy and stability of the whole process of binding site detection.

All of the above optional technical solutions are combined randomly to form optional embodiments of this disclosure. Details are not described herein again.

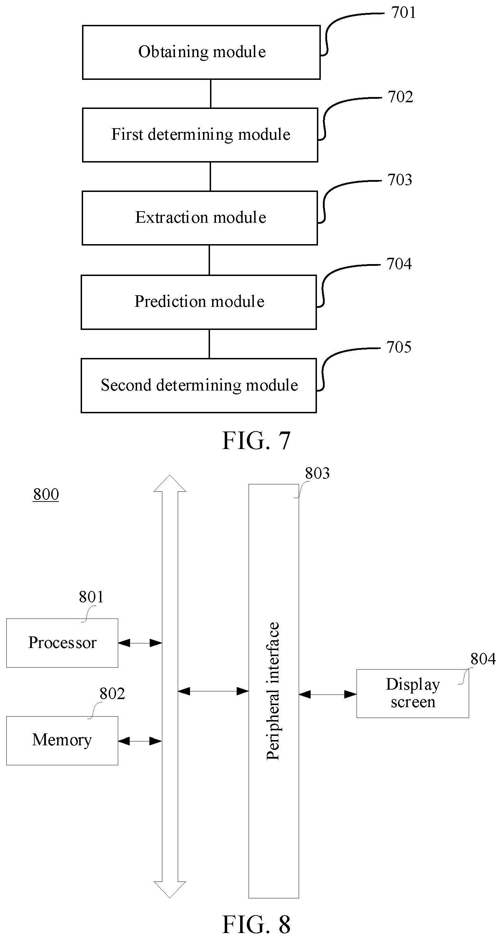

FIG. 7 is a schematic structural diagram of an apparatus for detecting a molecule binding site according to an embodiment of this disclosure. Referring to FIG. 7, the apparatus includes an obtaining module 701, a first determining module 702, an extraction module 703, a prediction module 704, and a second determining module 705.

The obtaining module 701 is configured to obtain 3D coordinates of at least one site in a target molecule, the target molecule being a chemical molecule with a to-be-detected binding site.

for each of the at least one site:

The first determining module 702 is configured to determine a first target point and a second target point, the first target point being a center point of all sites within a spherical space, the spherical space being a spherical space with the each of the at least one site as a center of a sphere and a target length as a radius, and the second target point being an intersection between a forward extension line of a vector, starting from an origin and pointing to the each of the at least one site, and an outer surface of the spherical space.

The extraction module 703 is configured to extract a rotation-invariant location feature in the 3D coordinates of the each of the at least one site based on the 3D coordinates of the each of the at least one site, 3D coordinates of the first target point, and 3D coordinates of the second target point, the rotation-invariant location feature being used for indicating location information of the each of the at least one site in the target molecule.

The prediction module 704 is configured to invoke a site detection model to perform prediction processing on the extracted rotation-invariant location feature, to obtain a prediction probability of the each of the at least one site, the each prediction probability indicating a possibility of the each of the at least one site being a binding site.

The second determining module 705 is configured to determine a binding site in the site in the target molecule based on the at least one prediction probability of the each of the at least one site.

In the apparatus provided in this embodiment of this disclosure, the 3D coordinates of each site in the target molecule are obtained, the first target point and the second target point corresponding to the each site are determined. Based on the 3D coordinates of the each site, the 3D coordinates of each first target point, and the 3D coordinates of each second target point, the rotation-invariant location feature in the 3D coordinates of the each site is extracted, and the site detection model is invoked to perform prediction on the extracted location feature, to obtain the prediction probability of the each site being a binding site, so as to determine the binding site of the target molecule based on the prediction probability. The first target point and the second target point are associated with each site and have spatial representativeness to some extent. Therefore, a rotation-invariant location feature that can completely reflect the detailed structure of the target molecule can be constructed based on the 3D coordinates of the each site, the 3D coordinates of each first target point, and the 3D coordinates of each second target point, thereby avoiding loss of details caused by designing a voxel feature for the target molecule, so that location information of the detailed structure of the target molecule can be fully used during binding site detection based on the location feature, thereby improving the accuracy of a process of detecting a molecule binding site.

In a possible implementation, based on the apparatus composition in FIG. 7, the extraction module 703 includes: an extraction unit, configured to extract, for any site in the at least one site, a rotation-invariant location feature in the 3D coordinates of the site based on the 3D coordinates of the site, 3D coordinates of the first target point that corresponds to the site, and 3D coordinates of the second target point that corresponds to the site.

In a possible implementation, the extraction unit is configured to:

construct a global location feature of the site based on the 3D coordinates of the site, the 3D coordinates of the first target point, and the 3D coordinates of the second target point, the global location feature being used for indicating spatial location information of the site in the target molecule;

construct, based on the 3D coordinates of the site, the 3D coordinates of the first target point, the 3D coordinates of the second target point, and 3D coordinates of at least one neighborhood point of the site, at least one local location feature between the site and the at least one neighborhood point, one local location feature being used for indicating relative location information between the site and one neighborhood point; and

obtain a location feature of the site based on the global location feature and the at least one local location feature.

In a possible embodiment, the global location feature includes at least one of a magnitude of the site, a distance between the site and the first target point, a distance between the first target point and the second target point, a cosine value of a first angle, or a cosine value of a second angle. The first angle is an angle formed between a first line segment and a second line segment, and the second angle is an angle formed between the second line segment and a third line segment. The first line segment is a line segment formed between the site and the first target point, the second line segment is a line segment formed between the first target point and the second target point, and the third line segment is a line segment formed between the site and the second target point.

In a possible embodiment, for any neighborhood point in the at least one neighborhood point, the local location feature between the site and the neighborhood point includes at least one of a distance between the neighborhood point and the site, a distance between the neighborhood point and the first target point, a distance between the neighborhood point and the second target point, a cosine value of a third angle, a cosine value of a fourth angle, or a cosine value of a fifth angle. The third angle is an angle formed between a fourth line segment and a fifth line segment, the fourth angle is an angle formed between the fifth line segment and a sixth line segment, and the fifth angle is an angle formed between the sixth line segment and the fourth line segment. The fourth line segment is a line segment formed between the neighborhood point and the site, the fifth line segment is a line segment formed between the neighborhood point and the first target point, and the sixth line segment is a line segment formed between the neighborhood point and the second target point.

In a possible implementation, the site detection model is a GCN; the GCN includes an input layer, at least one edge convolutional layer, and an output layer.

Based on the apparatus composition in FIG. 7, the prediction module 704 includes:

an input/output (I/O) unit, configured to input the location feature of the at least one site into the input layer in the GCN, and output graph data of the at least one site by using the input layer, the graph data being used for indicating the location feature of the site in the form of a graph;

a feature extraction unit, configured to input the graph data of the at least one site into the at least one edge convolutional layer in the GCN, and perform feature extraction on the graph data of the at least one site by using the at least one edge convolutional layer, to obtain a global biological feature of the at least one site; and

a probability fitting unit, configured to fuse the global biological feature, the graph data of the at least one site, and an edge convolutional feature outputted by the at least one edge convolutional layer, input a fused feature into the output layer of the GCN, and perform, by using the output layer, probability fitting on the fused feature, to obtain the at least one prediction probability.

In a possible implementation, the I/O unit is configured to:

input the location feature of the at least one site into an MLP in the input layer, and map the location feature of the at least one site by using the MLP, to obtain a first feature of the at least one site, a dimension quantity of the first feature being greater than a dimension quantity of the location feature; and

input the first feature of the at least one site into a pooling layer in the input layer, and perform dimension reduction on the first feature of the at least one site by using the pooling layer, to obtain the graph data of the at least one site.

In a possible implementation, based on the apparatus composition in FIG. 7, the feature extraction unit includes:

an extraction/input subunit, configured to perform, for any edge convolutional layer in the at least one edge convolutional layer, feature extraction on an edge convolutional feature outputted by a previous edge convolutional layer, and input an extracted edge convolutional feature into a next edge convolutional layer;

a concatenation subunit, configured to concatenate the graph data of the at least one site and at least one edge convolutional feature outputted by the at least one edge convolutional layer, to obtain a second feature;

a mapping subunit, configured to input the second feature into an MLP, and map the second feature by using the MLP, to obtain a third feature; and

a dimension reduction subunit, configured to input the third feature into a pooling layer, and perform dimension reduction on the third feature by using the pooling layer, to obtain the global biological feature.

In a possible implementation, the extraction/input subunit is configured to:

construct a cluster map for the any edge convolutional layer in the at least one edge convolutional layer based on the edge convolutional feature outputted by the previous edge convolutional layer;

input the cluster map into an MLP in the edge convolutional layer, and map the cluster map by using the MLP, to obtain an intermediate feature of the cluster map; and

input the intermediate feature into a pooling layer in the edge convolutional layer, perform dimension reduction on the intermediate feature by using the pooling layer, and input the dimension-reduced intermediate feature into the next edge convolutional layer.

In a possible implementation, the probability fitting unit is configured to:
input the fused feature into an MLP in the output layer, and map the fused feature by using the MLP, to obtain the at least one prediction probability.
In a possible implementation, the second determining module 705 is configured to:
determine a site with a prediction probability greater than a probability threshold from the at least one site as the binding site.
All of the above optional technical solutions are combined randomly to form optional embodiments of this disclosure. Details are not described herein again.
When the apparatus for detecting a molecule binding site provided in the foregoing embodiments detects a binding site in a target molecule, the division of the functional modules is merely used as an example for illustration. In the practical application, the functions may be allocated to and completed by different functional modules according to the requirements, that is, the internal structure of the electronic device is divided into different functional modules, to implement all or some of the functions described above. In addition, the apparatus for detecting a molecule binding site and the method for detecting a molecule binding site embodiments provided in the foregoing embodiments belong to one conception. For the specific implementation process, reference may be made to the embodiments of the method for detecting a molecule binding site, and details are not described herein again.

FIG. 8 is a schematic structural diagram of an electronic device according to an embodiment of this disclosure. Referring to FIG. 8, descriptions are made by using an example in which the electronic device is a terminal 800. The terminal 800 may be a smartphone, a tablet computer, a moving picture experts group audio layer III (MP3) player, a moving picture experts group audio layer IV (MP4) player, a notebook computer, or a desktop computer. The terminal 800 may also be referred to as user equipment, a portable terminal, a laptop terminal, a desktop terminal, or by another name.

Generally, the terminal 800 includes a processor 801 and a memory 802.

The processor 801 includes one or more processing cores, for example, a 4-core processor or an 8-core processor. The processor 801 may be implemented in at least one hardware form of a digital signal processor (DSP), a field-programmable gate array (FPGA), and a programmable logic array (PLA). In some embodiments, the processor 801 includes a main processor and a coprocessor. The main processor is a processor configured to process data in an awake state, and is also referred to as a central processing unit (CPU). The coprocessor is a low-power processor configured to process data in a standby state. In some embodiments, a GPU is integrated with the processor 801. The GPU is configured to be responsible for rendering and drawing content to be displayed on a display screen. In some embodiments, the processor 801 includes an artificial intelligence (AI) processor. The AI processor is configured to process a computing operation related to machine learning.

The memory 802 includes one or more non-transitory computer-readable storage media. The computer-readable storage medium is non-transient. In some embodiments, the memory 802 further includes a high-speed random access memory and a nonvolatile memory, for example, one or more disk storage devices or flash storage devices. In some embodiments, the non-transitory computer-readable storage medium in the memory 802 is configured to store at least one instruction, and the at least one instruction is configured to be executed by the processor 801 to implement the following steps of detecting a molecule binding site:

obtaining 3D coordinates of at least one site in a target molecule to be detected, the target molecule being a chemical molecule with a binding site to be detected;

respectively determining a first target point and a second target point corresponding to each site, the first target point of any site being a center point of all sites within a target spherical space, the target spherical space being a spherical space with the any site as a center of a sphere and a target length as a radius, and the second target point of any site being an intersection between a forward extension line of a vector, starting from an origin and pointing to the site, and an outer surface of the target spherical space;

extracting a rotation-invariant location feature in the 3D coordinates of the at least one site based on the 3D coordinates of the at least one site, 3D coordinates of at least one first target point, and 3D coordinates of at least one second target point, the location feature being used for indicating location information of the at least one site in the target molecule;

invoking a site detection model to perform prediction processing on the extracted location feature, to obtain at least one prediction probability of the at least one site, each prediction probability being used for indicating a possibility of a site being a binding site; and

determining a binding site in the at least one site in the target molecule based on the at least one prediction probability.

In a possible implementation, the extracting a rotation-invariant location feature in the 3D coordinates of the at least one site based on the 3D coordinates of the at least one site, 3D coordinates of at least one first target point, and 3D coordinates of at least one second target point includes:
extracting, for any site in the at least one site, a rotation-invariant location feature in the 3D coordinates of the site based on the 3D coordinates of the site, 3D coordinates of the first target point that corresponds to the site, and 3D coordinates of the second target point that corresponds to the site.

In a possible implementation, the extracting a rotation-invariant location feature in 3D coordinates of the site based on the 3D coordinates of the site, 3D coordinates of the first target point that corresponds to the site, and 3D coordinates of the second target point that corresponds to the site includes:

constructing a global location feature of the site based on the 3D coordinates of the site, the 3D coordinates of the first target point, and the 3D coordinates of the second target point, the global location feature being used for indicating spatial location information of the site in the target molecule;

constructing, based on the 3D coordinates of the site, the 3D coordinates of the first target point, the 3D coordinates of the second target point, and 3D coordinates of at least one neighborhood point of the site, at least one local location feature between the site and the at least one neighborhood point, one local location feature being used for indicating relative location information between the site and one neighborhood point; and

obtaining a location feature of the site based on the global location feature and the at least one local location feature.

In a possible embodiment, the global location feature includes at least one of a magnitude of the site, a distance between the site and the first target point, a distance between the first target point and the second target point, a cosine value of a first angle, or a cosine value of a second angle. The first angle is an angle formed between a first line segment and a second line segment, and the second angle is an angle formed between the second line segment and a third line segment. The first line segment is a line segment formed between the site and the first target point, the second line segment is a line segment formed between the first target point and the second target point, and the third line segment is a line segment formed between the site and the second target point.

In a possible embodiment, for any neighborhood point in the at least one neighborhood point, the local location feature between the site and the neighborhood point includes at least one of a distance between the neighborhood point and the site, a distance between the neighborhood point and the first target point, a distance between the neighborhood point and the second target point, a cosine value of a third angle, a cosine value of a fourth angle, or a cosine value of a fifth angle. The third angle is an angle formed between a fourth line segment and a fifth line segment, the fourth angle is an angle formed between the fifth line segment and a sixth line segment, and the fifth angle is an angle formed between the sixth line segment and the fourth line segment. The fourth line segment is a line segment formed between the neighborhood point and the site, the fifth line segment is a line segment formed between the neighborhood point and the first target point, and the sixth line segment is a line segment formed between the neighborhood point and the second target point.

In a possible implementation, the site detection model is a GCN; the GCN includes an input layer, at least one edge convolutional layer, and an output layer.

The invoking a site detection model to perform prediction on the extracted location feature, to obtain at least one prediction probability of the at least one site includes:

inputting the location feature of the at least one site into the input layer in the GCN, and outputting graph data of the at least one site by using the input layer, the graph data being used for indicating the location feature of the site in the form of a graph;

inputting the graph data of the at least one site into the at least one edge convolutional layer in the GCN, and performing feature extraction on the graph data of the at least one site by using the at least one edge convolutional layer, to obtain a global biological feature of the at least one site; and

fusing the global biological feature, the graph data of the at least one site, and an edge convolutional feature outputted by the at least one edge convolutional layer, inputting a fused feature into the output layer of the GCN, and performing, by using the output layer, probability fitting on the fused feature, to obtain the at least one prediction probability.

In a possible implementation, the inputting the location feature of the at least one site into the input layer in the GCN, and outputting graph data of the at least one site by using the input layer includes:

inputting the location feature of the at least one site into an MLP in the input layer, and mapping the location feature of the at least one site by using the MLP, to obtain a first feature of the at least one site, a dimension quantity of the first feature being greater than a dimension quantity of the location feature; and

inputting the first feature of the at least one site into a pooling layer in the input layer, and performing dimension reduction on the first feature of the at least one site by using the pooling layer, to obtain the graph data of the at least one site.

In a possible implementation, the performing feature extraction on the graph data of the at least one site by using the at least one edge convolutional layer, to obtain a global biological feature of the at least one site includes:

performing, for any edge convolutional layer in the at least one edge convolutional layer, feature extraction on an edge convolutional feature outputted by a previous edge convolutional layer, and inputting an extracted edge convolutional feature into a next edge convolutional layer;

concatenating the graph data of the at least one site and at least one edge convolutional feature outputted by the at least one edge convolutional layer, to obtain a second feature;

inputting the second feature into an MLP, and mapping the second feature by using the MLP, to obtain a third feature; and

inputting the third feature into a pooling layer, and performing dimension reduction on the third feature by using the pooling layer, to obtain the global biological feature.

In a possible implementation, the performing, for any edge convolutional layer in the at least one edge convolutional layer, feature extraction on an edge convolutional feature outputted by a previous edge convolutional layer, and inputting an extracted edge convolutional feature into a next edge convolutional layer includes:

constructing a cluster map for the any edge convolutional layer in the at least one edge convolutional layer based on the edge convolutional feature outputted by the previous edge convolutional layer;

inputting the cluster map into an MLP in the edge convolutional layer, and mapping the cluster map by using the MLP, to obtain an intermediate feature of the cluster map; and

inputting the intermediate feature into a pooling layer in the edge convolutional layer, performing dimension reduction on the intermediate feature by using the pooling layer, and inputting the dimension-reduced intermediate feature into the next edge convolutional layer.

In a possible implementation, the inputting a fused feature into the output layer of the GCN, and performing, by using the output layer, probability fitting on the fused feature, to obtain the at least one prediction probability includes: inputting the fused feature into an MLP in the output layer, and mapping the fused feature by using the MLP, to obtain the at least one prediction probability.

In a possible implementation, the determining a binding site in the at least one site in the target molecule based on the at least one prediction probability includes: determining a site with a prediction probability greater than a probability threshold from the at least one site as the binding site.

In some embodiments, the terminal 800 may alternatively include: a peripheral interface 803 and at least one peripheral. The processor 801, the memory 802, and the peripheral interface 803 may be connected through a bus or a signal cable. Each peripheral is connected to the peripheral interface 803 through a bus, a signal cable, or a circuit board. Optionally, the peripheral includes a display screen 804.

The peripheral interface 803 may be configured to connect at least one peripheral device related to I/O to the processor 801 and the memory 802.

The display screen 804 is configured to display a user interface (UI). The UI may include a graph, a text, an icon, a video, and any combination thereof. In a case that the display screen 804 is a touch display screen, the display screen 804 further has a capability of acquiring a touch signal on or above a surface of the display screen 804. In some embodiments, the touch signal may be inputted to the processor 801 for processing as a control signal. In this case, the display screen 804 is further configured to provide a virtual button and/or a virtual keyboard, which is also referred to as a soft button and/or a soft keyboard.

A person skilled in the art can understand that the structure shown in FIG. 8 does not constitute a limitation to the terminal 800, and the terminal may include more or fewer components than those shown in the figure, or some components may be combined, or a different component arrangement may be used.

In an exemplary embodiment, a non-transitory computer-readable storage medium, for example, a memory including at least one piece of program code, is further provided. The at least one piece of program code may be executed by the processor in the terminal to implement the following molecule binding-site detection steps:

obtaining 3D coordinates of at least one site in a target molecule to be detected, the target molecule being a chemical molecule with a binding site to be detected;

respectively determining a first target point and a second target point corresponding to each site, the first target point of any site being a center point of all sites within a target spherical space, the target spherical space being a spherical space with the any site as a center of a sphere and a target length as a radius, and the second target point of any site being an intersection between a forward extension line of a vector, starting from an origin and pointing to the site, and an outer surface of the target spherical space;

extracting a rotation-invariant location feature in the 3D coordinates of the at least one site based on the 3D coordinates of the at least one site, 3D coordinates of at least one first target point, and 3D coordinates of at least one second target point, the location feature being used for indicating location information of the at least one site in the target molecule;

invoking a site detection model to perform prediction on the extracted location feature, to obtain at least one prediction probability of the at least one site, each prediction probability being used for indicating a possibility of a site being a binding site; and

determining a binding site in the at least one site in the target molecule based on the at least one prediction probability.

In a possible implementation, the extracting a rotation-invariant location feature in the 3D coordinates of the at least one site based on the 3D coordinates of the at least one site, 3D coordinates of at least one first target point, and 3D coordinates of at least one second target point includes:

extracting, for any site in the at least one site, a rotation-invariant location feature in the 3D coordinates of the site based on the 3D coordinates of the site, 3D coordinates of the first target point that corresponds to the site, and 3D coordinates of the second target point that corresponds to the site.

In a possible implementation, the extracting a rotation-invariant location feature in the 3D coordinates of the site based on the 3D coordinates of the site, 3D coordinates of the first target point that corresponds to the site, and 3D coordinates of the second target point that corresponds to the site includes:

constructing a global location feature of the site based on the 3D coordinates of the site, the 3D coordinates of the first target point, and the 3D coordinates of the second target point, the global location feature being used for indicating spatial location information of the site in the target molecule;

constructing, based on the 3D coordinates of the site, the 3D coordinates of the first target point, the 3D coordinates of the second target point, and 3D coordinates of at least one neighborhood point of the site, at least one local location feature between the site and the at least one neighborhood point, one local location feature being used for indicating relative location information between the site and one neighborhood point; and

obtaining a location feature of the site based on the global location feature and the at least one local location feature.

In a possible embodiment, the global location feature includes at least one of a magnitude of the site, a distance between the site and the first target point, a distance between the first target point and the second target point, a cosine value of a first angle, or a cosine value of a second angle. The first angle is an angle formed between a first line segment and a second line segment, and the second angle is an angle formed between the second line segment and a third line segment. The first line segment is a line segment formed between the site and the first target point, the second line segment is a line segment formed between the first target point and the second target point, and the third line segment is a line segment formed between the site and the second target point.

In a possible embodiment, for any neighborhood point in the at least one neighborhood point, the local location feature between the site and the neighborhood point includes at least one of a distance between the neighborhood point and the site, a distance between the neighborhood point and the first target point, a distance between the neighborhood point and the second target point, a cosine value of a third angle, a cosine value of a fourth angle, or a cosine value of a fifth angle. The third angle is an angle formed between a fourth line segment and a fifth line segment, the fourth angle is an angle formed between the fifth line segment and a sixth line segment, and the fifth angle is an angle formed between the sixth line segment and the fourth line segment. The fourth line segment is a line segment formed between the neighborhood point and the site, the fifth line segment is a line segment formed between the neighborhood point and the first target point, and the sixth line segment is a line segment formed between the neighborhood point and the second target point.

In a possible implementation, the site detection model is a GCN; the GCN includes an input layer, at least one edge convolutional layer, and an output layer.

The invoking a site detection model to perform prediction processing on the extracted location feature, to obtain at least one prediction probability of the at least one site includes:

inputting the location feature of the at least one site into the input layer in the GCN, and outputting graph data of the at least one site by using the input layer, the graph data being used for indicating the location feature of the site in the form of a graph;

inputting the graph data of the at least one site into the at least one edge convolutional layer in the GCN, and performing feature extraction on the graph data of the at least one site by using the at least one edge convolutional layer, to obtain a global biological feature of the at least one site; and

fusing the global biological feature, the graph data of the at least one site, and an edge convolutional feature outputted by the at least one edge convolutional layer, inputting a fused feature into the output layer of the GCN, and performing, by using the output layer, probability fitting on the fused feature, to obtain the at least one prediction probability.

In a possible implementation, the inputting the location feature of the at least one site into the input layer in the GCN, and outputting graph data of the at least one site by using the input layer includes:

inputting the location feature of the at least one site into an MLP in the input layer, and mapping the location feature of the at least one site by using the MLP, to obtain a first feature of the at least one site, a dimension quantity of the first feature being greater than a dimension quantity of the location feature; and

inputting the first feature of the at least one site into a pooling layer in the input layer, and performing dimension reduction on the first feature of the at least one site by using the pooling layer, to obtain the graph data of the at least one site.

In a possible implementation, the performing feature extraction on the graph data of the at least one site by using the at least one edge convolutional layer, to obtain a global biological feature of the at least one site includes:

performing, for any edge convolutional layer in the at least one edge convolutional layer, feature extraction on an edge convolutional feature outputted by a previous edge convolutional layer, and inputting an extracted edge convolutional feature into a next edge convolutional layer;

concatenating the graph data of the at least one site and at least one edge convolutional feature outputted by the at least one edge convolutional layer, to obtain a second feature;

inputting the second feature into an MLP, and mapping the second feature by using the MLP, to obtain a third feature; and

inputting the third feature into a pooling layer, and performing dimension reduction on the third feature by using the pooling layer, to obtain the global biological feature.

In a possible implementation, the performing, for any edge convolutional layer in the at least one edge convolutional layer, feature extraction on an edge convolutional feature outputted by a previous edge convolutional layer, and inputting an extracted edge convolutional feature into a next edge convolutional layer includes:

constructing a cluster map for the any edge convolutional layer in the at least one edge convolutional layer based on the edge convolutional feature outputted by the previous edge convolutional layer;
inputting the cluster map into an MLP in the edge convolutional layer, and mapping the cluster map by using the MLP, to obtain an intermediate feature of the cluster map; and
inputting the intermediate feature into a pooling layer in the edge convolutional layer, performing dimension reduction on the intermediate feature by using the pooling layer, and inputting the dimension-reduced intermediate feature into the next edge convolutional layer.

In a possible implementation, the inputting a fused feature into the output layer of the GCN, and performing, by using the output layer, probability fitting on the fused feature, to obtain the at least one prediction probability includes:
inputting the fused feature into an MLP in the output layer, and mapping the fused feature by using the MLP, to obtain the at least one prediction probability.
In a possible implementation, the determining a binding site in the at least one site in the target molecule based on the at least one prediction probability includes:
determining a site with a prediction probability greater than a probability threshold from the at least one site as the binding site.

In some embodiments, the non-transitory computer-readable storage medium may be a read-only memory (ROM), a random access memory (RAM), a compact disc read-only memory (CD-ROM), a magnetic tape, a floppy disk, an optical data storage device, or the like.

A person of ordinary skill in the art can understand that all or some of the steps of the embodiments may be implemented by hardware or a program instructing related hardware. The program is stored in a non-transitory computer-readable storage medium. The non-transitory storage medium includes a read-only memory, a magnetic disk, or an optical disc.

**Claims**

1. A computer-implemented method for detecting a molecule binding site, applicable to an electronic device, the method comprising:

   obtaining (201) three-dimensional 3D coordinates of at least one site in a target molecule, the target molecule being a chemical molecule with a to-be-detected binding site;
   for each site of the at least one site:

   determining (202) a first target point and a second target point, the first target point being a center point of all sites within a spherical space, the spherical space being a spherical space with the respective site as a center of a sphere and a target length as a radius, and the second target point being an intersection between a forward extension line of a vector, starting from an origin and pointing to the respective site, and an outer surface of the spherical space, the origin being an origin of a 3D coordinate system in which the target molecule is located, and
   extracting (203) a rotation-invariant location feature in the 3D coordinates of the respective site based on the 3D coordinates of the respective site, 3D coordinates of the first target point, and 3D coordinates of the second target point, the rotation-invariant location feature being used for indicating location information of the respective site in the target molecule;

   invoking (204) a site detection model to perform prediction processing on the extracted rotation-invariant location feature, to obtain a prediction probability of the each site of the at least one site, the each prediction probability indicating a possibility of the each site of the at least one site being a binding site;
   determining (205) a binding site from the at least one site in the target molecule based on the prediction probability of the each site of the at least one site;
   wherein the extracting the rotation-invariant location feature in the 3D coordinates of the respective site comprises:

   constructing (302) a global location feature of the respective site based on the 3D coordinates of the respective site, the 3D coordinates of the first target point, and the 3D coordinates of the second target point, the global location feature being used for indicating spatial location information of the site in the target molecule;
   constructing (303), based on the 3D coordinates of the respective site, the 3D coordinates of the first target point, the 3D coordinates of the second target point, and 3D coordinates of at least one neighborhood point of the site, at least one local location feature between the site and the at least one neighborhood point, the at least one local location feature being used for indicating relative location information between the respective site and the at least one neighborhood point; and
   obtaining (304) the location feature of the respective site based on the global location feature and the at least one local location feature.

2. The method according to claim 1, wherein the global location feature comprises at least one of a magnitude of the respective site, a distance between the respective site and the first target point, a distance between the first target point and the second target point, a cosine value of a first angle, or a cosine value of a second angle, the first angle being an angle formed between a first line segment and a second line segment, the second angle being an angle formed between the second line segment and a third line segment, the first line segment being a line segment formed between the respective site and the first target point, the second line segment being a line segment formed between the first target point and the second target point, and the third line segment being a line segment formed between the respective site and the second target point.

3. The method according to claim 1, wherein for any neighborhood point in the at least one neighborhood point, the local location feature between the respective site and the neighborhood point comprises at least one of a distance between the neighborhood point and the site, a distance between the neighborhood point and the first target point, a distance between the neighborhood point and the second target point, a cosine value of a third angle, a cosine value of a fourth angle, or a cosine value of a fifth angle, the third angle being an angle formed between a fourth line segment and a fifth line segment, the fourth angle being an angle formed between the fifth line segment and a sixth line segment, the fifth angle being an angle formed between the sixth line segment and the fourth line segment, the fourth line segment being a line segment formed between the neighborhood point and the respective site, the fifth line segment being a line segment formed between the neighborhood point and the first target point, and the sixth line segment being a line segment formed between the neighborhood point and the second target point.

4. The method according to claim 1, wherein the site detection model is a graph convolutional network, GCN, and the GCN comprises an input layer, at least one edge convolutional layer, and an output layer; and
the invoking the site detection model to perform prediction processing on the extracted rotation-invariant location feature, to obtain the prediction probability of the each site of the at least one site comprises:

inputting (306) the location feature of the each site of the at least one site into the input layer of the GCN, and outputting graph data of the each site of the at least one site by using the input layer, the graph data being used for indicating the location feature of the each site of the at least one site in the form of a graph;
inputting (307) the graph data of the each site of the at least one site into the at least one edge convolutional layer of the GCN, and performing feature extraction on the graph data of the each site of the at least one site by using the at least one edge convolutional layer, to obtain a global biological feature of the each site of the at least one site;
fusing (308) the global biological feature, the graph data of the each site of the at least one site, and an edge convolutional feature outputted by the at least one edge convolutional layer to obtain a fused feature;
inputting (308) the fused feature into the output layer of the GCN, and performing, by using the output layer, probability fitting on the fused feature, to obtain the prediction probability.

5. The method according to claim 4, wherein inputting the location feature of the each site of the at least one site into the input layer of the GCN, and outputting graph data of the each site of the at least one site by using the input layer comprises:

inputting the location feature of the each site of the at least one site into a multilayer perceptron, MLP of the input layer, and mapping the location feature of the each site of the at least one site by using the MLP, to obtain a first feature of the each site of the at least one site, a dimension quantity of the first feature being greater than a dimension quantity of the location feature; and
inputting the first feature of the each site of the at least one site into a pooling layer of the input layer, and performing dimension reduction on the first feature of the each site of the least one site by using the pooling layer, to obtain the graph data of the each site of the at least one site.

6. The method according to claim 4, wherein performing the feature extraction on the graph data of the each site of the at least one site by using the at least one edge convolutional layer, to obtain a global biological feature of the each site of the at least one site comprises:

performing, for each edge convolutional layer in the at least one edge convolutional layer, feature extraction on an edge convolutional feature outputted by a previous edge convolutional layer, and inputting the extracted edge convolutional feature into a next edge convolutional layer;
concatenating the graph data of the each site of the at least one site and at least one edge convolutional feature outputted by the at least one edge convolutional layer, to obtain a second feature;
inputting the second feature into a multilayer perceptron, MLP, and mapping the second feature by using the MLP, to obtain a third feature; and
inputting the third feature into a pooling layer, and performing dimension reduction on the third feature by using the pooling layer, to obtain the global biological feature.

7. The method according to claim 6, wherein performing, for the each edge convolutional layer in the at least one edge convolutional layer, the feature extraction on an edge convolutional feature outputted by a previous edge convolutional layer, and inputting the extracted edge convolutional feature into the next edge convolutional layer comprises:

constructing a cluster map for the each edge convolutional layer in the at least one edge convolutional layer based on the edge convolutional feature outputted by the previous edge convolutional layer;

inputting the cluster map into an MLP of the edge convolutional layer, and mapping the cluster map by using the MLP, to obtain an intermediate feature of the cluster map; and

inputting the intermediate feature into a pooling layer in the edge convolutional layer, performing dimension reduction on the intermediate feature by using the pooling layer, and inputting the dimension-reduced intermediate feature into the next edge convolutional layer.

8. The method according to claim 4, wherein inputting the fused feature into the output layer of the GCN, and performing, by using the output layer, probability fitting on the fused feature, to obtain the prediction probability comprises:

inputting the fused feature into a multilayer perceptron, MLP, in the output layer, and mapping the fused feature by using the MLP, to obtain the prediction probability.

9. The method according to claim 1, wherein determining the binding site from the at least one site in the target molecule based on the prediction probability of the each site of the at least one site comprises:

determining a site with a prediction probability greater than a probability threshold from the at least one site as the binding site.

10. An apparatus for detecting a molecule binding site, comprising:

an obtaining module (701), configured to obtain three-dimensional 3Dcoordinates of at least one site in a target molecule, the target molecule being a chemical molecule with a to-be-detected binding site;
for each site of the at least one site:

a first determining module (702), configured to determine a first target point and a second target point, the first target point being a center point of all sites within a spherical space, the spherical space being a spherical space with the respective site as a center of a sphere and a target length as a radius, and the second target point being an intersection between a forward extension line of a vector, starting from an origin and pointing to the respective site, and an outer surface of the spherical space, the origin being an origin of a 3D coordinate system in which the target molecule is located, and

an extraction module (703), configured to extract a rotation-invariant location feature in the 3D coordinates of the respective site based on the 3D coordinates of the respective site, 3D coordinates of the first target point, and 3D coordinates of the second target point, the rotation-invariant location feature being used for indicating location information of the respective site in the target molecule;

a prediction module (704), configured to invoke a site detection model to perform prediction on the extracted rotation-invariant location feature, to obtain a prediction probability of the each site of the at least one site, the each prediction probability indicating a possibility of the each site of the at least one site being a binding site; and
a second determining module (705), configured to determine a binding site from the at least one site in the target molecule based on the prediction probability of the each site of the at least one site.

11. An electronic device, comprising one or more processors and one or more memories, the one or more memories storing at least one piece of program code, the at least one piece of program code being loaded and executed by the one or more processors to implement the method for detecting a molecule binding site according to any one of claims 1 to 9.

12. A storage medium, storing at least one piece of program code, the at least one piece of program code being loaded and executed by a processor to implement method for detecting a molecule binding site according to any one of claims 1 to 9.

## Patentansprüche

1. Computerimplementiertes Verfahren zum Detektieren einer Molekülbindungsstelle, das auf eine elektronische Vorrichtung anwendbar ist, wobei das Verfahren Folgendes umfasst:

Erhalten (201) von dreidimensionalen 3D-Koordinaten von mindestens einer Stelle in einem Zielmolekül, wobei das Zielmolekül ein chemisches Molekül mit einer zu detektierenden Bindungsstelle ist;
für jede Stelle der mindestens einen Stelle:

Bestimmen (202) eines ersten Zielpunkts und eines zweiten Zielpunkts, wobei der erste Zielpunkt einen Mittelpunkt von allen Stellen in einem Kugelraum ist, wobei der Kugelraum ein Kugelraum mit der jeweiligen Stelle als einer Mitte einer Kugel und einer Ziellänge als einem Radius ist, und wobei der zweite Zielpunkt eine Überschneidung zwischen einer Vorwärtserstreckungslinie eines Vektors, beginnend bei einem Ursprung und auf die jeweilige Stelle zeigend, und einer Außenfläche des Kugelraums ist, wobei der Ursprung ein Ursprung eines 3D-Koordinatensystems ist, in dem sich das Zielmolekül befindet, und

Extrahieren (203) eines rotationsinvarianten Ortsmerkmals in den 3D-Koordinaten der jeweiligen Stelle auf Basis der 3D-Koordinaten der jeweiligen Stelle, 3D-Koordinaten des ersten Zielpunkts und 3D-Koordinaten des zweiten Zielpunkts, wobei das rotationsinvariante Ortsmerkmal zum Anzeigen von Ortsinformationen der jeweiligen Stelle im Zielmolekül verwendet wird;

Aufrufen (204) eines Stellendetektionsmodells, um eine Vorhersageverarbeitung am extrahierten rotationsinvarianten Ortsmerkmal durchzuführen, um eine Vorhersagewahrscheinlichkeit jeder Stelle der mindestens einen Stelle zu erhalten, wobei jede Vorhersagewahrscheinlichkeit eine Wahrscheinlichkeit anzeigt, dass jede Stelle der mindestens einen Stelle eine Bindungsstelle ist;

Bestimmen (205) einer Bindungsstelle der mindestens einen Stelle im Zielmolekül auf Basis der Vorhersagewahrscheinlichkeit jeder Stelle der mindestens einen Stelle;

wobei das Extrahieren des rotationsinvarianten Ortsmerkmals in den 3D-Koordinaten der jeweiligen Stelle Folgendes umfasst:

Konstruieren (302) eines globalen Ortsmerkmals der jeweiligen Stelle auf Basis der 3D-Koordinaten der jeweiligen Stelle, der 3D-Koordinaten des ersten Zielpunkts und der 3D-Koordinaten des zweiten Zielpunkts, wobei das globale Ortsmerkmal zum Anzeigen von räumlichen Ortsinformationen der Stelle im Zielmolekül verwendet wird;

Konstruieren (303) von mindestens einem lokalen Ortsmerkmal zwischen der Stelle und mindestens einem Nachbarpunkt auf Basis der 3D-Koordinaten der jeweiligen Stelle, der 3D-Koordinaten des ersten Zielpunkts, der 3D-Koordinaten des zweiten Zielpunkts und 3D-Koordinaten des mindestens einen Nachbarpunkts der Stelle, wobei das mindestens eine lokale Ortsmerkmal zum Anzeigen von relativen Ortsinformationen zwischen der jeweiligen Stelle und dem mindestens einen Nachbarpunkt verwendet wird; und

Erhalten (304) des Ortsmerkmals der jeweiligen Stelle auf Basis des globalen Ortsmerkmals und des mindestens einen lokalen Ortsmerkmals.

2. Verfahren nach Anspruch 1, wobei das globale Ortsmerkmal mindestens eines von einer Größe der jeweiligen Stelle, einem Abstand zwischen der jeweiligen Stelle und dem ersten Zielpunkt, einem Abstand zwischen dem ersten Zielpunkt und dem zweiten Zielpunkt, einem Kosinuswert eines ersten Winkels oder einem Kosinuswert eines zweiten Winkels umfasst, wobei der erste Winkel ein Winkel ist, der zwischen einem ersten Liniensegment und einem zweiten Liniensegment gebildet ist, wobei der zweite Winkel ein Winkel ist, der zwischen dem zweiten Liniensegment und einem dritten Liniensegment gebildet ist, wobei das erste Liniensegment ein Liniensegment ist, das zwischen der jeweiligen Stelle und dem ersten Zielpunkt gebildet ist, wobei das zweite Liniensegment ein Liniensegment ist, das zwischen dem ersten Zielpunkt und dem zweiten Zielpunkt gebildet ist, und wobei das dritte Liniensegment ein Liniensegment ist, das zwischen der jeweiligen Stelle und dem zweiten Zielpunkt gebildet ist.

3. Verfahren nach Anspruch 1, wobei für jeden Nachbarpunkt des mindestens einen Nachbarpunkts das lokale Ortsmerkmal zwischen der jeweiligen Stelle und dem Nachbarpunkt mindestens eines von einem Abstand zwischen dem Nachbarpunkt und der Stelle, einem Abstand zwischen dem Nachbarpunkt und dem ersten Zielpunkt, einem Abstand zwischen dem Nachbarpunkt und dem zweiten Zielpunkt, einem Kosinuswert eines dritten Winkels, einem Kosinuswert eines vierten Winkels oder einem Kosinuswert eines fünften Winkels umfasst, wobei der dritte Winkel ein Winkel ist, der zwischen einem vierten Liniensegment und einem fünften Liniensegment gebildet ist, wobei der vierte Winkel ein Winkel ist, der zwischen dem fünften Liniensegment und einem sechsten Liniensegment gebildet ist, wobei der fünfte Winkel ein Winkel ist, der zwischen dem sechsten Liniensegment und dem vierten Liniensegment gebildet ist, wobei das vierte Liniensegment ein Liniensegment ist, das zwischen dem Nachbarpunkt und der jeweiligen Stelle gebildet ist, wobei das fünfte Liniensegment ein Liniensegment ist, das zwischen dem Nachbarpunkt und dem ersten Zielpunkt gebildet ist, und wobei das sechste Liniensegment ein Liniensegment ist, das zwischen dem Nachbarpunkt und dem zweiten Zielpunkt gebildet ist.

4. Verfahren nach Anspruch 1, wobei das Stellendetektionsmodell ein Graphenfaltungsnetzwerk, GCN, ist und das GCN eine Eingangsschicht, mindestens eine Edgefaltungsschicht und eine Ausgangsschicht umfasst; und
das Aufrufen des Stellendetektionsmodells, um eine Vorhersageverarbeitung am extrahierten rotationsinvarianten Ortsmerkmal durchzuführen, um die Vorhersagewahrscheinlichkeit jeder Stelle der mindestens einen Stelle zu erhalten, umfasst Folgendes:

Eingeben (306) des Ortsmerkmals von jeder Stelle der mindestens einen Stelle in die Eingangsschicht des GCN und Ausgeben von Graphendaten jeder Stelle der mindestens einen Stelle unter Verwendung der Eingangsschicht, wobei die Graphendaten zum Anzeigen des Ortsmerkmals jeder Stelle der mindestens einen Stelle in Form eines Graphen verwendet werden;

Eingeben (307) der Graphendaten jeder Stelle der mindestens einen Stelle in die mindestens eine Edgefaltungsschicht des GCN und Durchführen einer Merkmalsextraktion an den Graphendaten jeder Stelle der mindestens einen Stelle unter Verwendung der mindestens einen Edgefaltungsschicht, um ein globales biologisches Merkmal jeder Stelle der mindestens einen Stelle zu erhalten;

Fusionieren (308) des globalen biologischen Merkmals, der Graphendaten jeder Stelle der mindestens einen Stelle und eines Edgefaltungsmerkmals, das von der mindestens einen Edgefaltungsschicht ausgegeben wird, um ein fusioniertes Merkmal zu erhalten;

Eingeben (308) des fusionierten Merkmals in die Ausgangsschicht des GCN und Durchführen eines Wahrscheinlichkeitseinsetzens unter Verwendung der Ausgangsschicht am fusionierten Merkmal, um die Vorhersagewahrscheinlichkeit zu erhalten.

5. Verfahren nach Anspruch 4, wobei das Eingeben des Ortsmerkmals jeder Stelle der mindestens einen Stelle in die Eingangsschicht des GCN und das Ausgeben von Graphendaten jeder Stelle der mindestens einen Stelle unter Verwendung der Eingangsschicht Folgendes umfasst:

Eingeben des Ortsmerkmals jeder Stelle der mindestens einen Stelle in ein mehrschichtiges Perzeptron, MLP, der Eingangsschicht und Zuordnen des Ortsmerkmals jeder Stelle der mindestens einen Stelle unter Verwendung des MLP, um ein erstes Merkmal jeder Stelle der mindestens einen Stelle zu erhalten, wobei eine Dimensionsmenge des ersten Merkmals größer ist als eine Dimensionsmenge des Ortsmerkmals; und

Eingeben des ersten Merkmals jeder Stelle der mindestens einen Stelle in eine Poolingschicht der Eingangsschicht und Durchführen einer Dimensionsreduzierung am ersten Merkmal jeder Stelle der mindestens einen Stelle unter Verwendung der Poolingschicht, um die Graphendaten jeder Stelle der mindestens einen Stelle zu erhalten.

6. Verfahren nach Anspruch 4, wobei das Durchführen der Merkmalsextraktion an den Graphendaten jeder Stelle der mindestens einen Stelle unter Verwendung der mindestens einen Edgefaltungsschicht, um ein globales biologisches Merkmal jeder Stelle der mindestens einen Stelle zu erhalten, Folgendes umfasst:

Durchführen einer Merkmalsextraktion an einem Edgefaltungsmerkmal, das von einer vorherigen Edgefaltungsschicht ausgegeben wurde, und Eingeben des extrahierten Edgefaltungsmerkmals in eine nächste Edgefaltungsschicht für jede Edgefaltungsschicht der mindestens einen Edgefaltungsschicht;

Verketten der Graphendaten jeder Stelle der mindestens einen Stelle und mindestens eines Edgefaltungsmerkmals, das von der mindestens einen Edgefaltungsschicht ausgegeben wird, um ein zweites Merkmal zu erhalten;

Eingeben des zweiten Merkmals in ein mehrschichtiges Perzeptron, MLP, und Zuordnen des zweiten Merkmals unter Verwendung des MLP, um ein drittes Merkmal zu erhalten; und

Eingeben des dritten Merkmals in eine Poolingschicht und Durchführen einer Dimensionsreduzierung am dritten Merkmal unter Verwendung der Poolingschicht, um das globale biologische Merkmal zu erhalten.

7. Verfahren nach Anspruch 6, wobei das Durchführen der Merkmalsextraktion an einem Edgefaltungsmerkmal, das von einer vorherigen Edgefaltungsschicht ausgegeben wurde, und das Eingeben des extrahierten Edgefaltungsmerkmals in die nächste Edgefaltungsschicht für jede Edgefaltungsschicht der mindestens einen Edgefaltungsschicht Folgendes umfasst:

Konstruieren einer Clusterkarte für jede Edgefaltungsschicht der mindestens einen Edgefaltungsschicht auf Basis des Edgefaltungsmerkmals, das von der vorherigen Edgefaltungsschicht ausgegeben wurde;

Eingeben der Clusterkarte in ein MLP der Edgefaltungsschicht und Zuordnen der Clusterkarte unter Verwendung des MLP, um ein Zwischenmerkmal der Clusterkarte zu erhalten; und

Eingeben des Zwischenmerkmals in eine Poolingschicht in der Edgefaltungsschicht, Durchführen einer Dimensionsreduzierung am Zwischenmerkmal unter Verwendung der Poolingschicht und Eingeben des dimensionsreduzierten Zwischenmerkmals in die nächste Edgefaltungsschicht.

8. Verfahren nach Anspruch 4, wobei das Eingeben des fusionierten Merkmals in die Ausgangsschicht des GCN und das Durchführen eines Wahrscheinlichkeitseinsetzens unter Verwendung der Ausgangsschicht am fusionierten Merkmal, um die Vorhersagewahrscheinlichkeit zu erhalten, Folgendes umfasst:
Eingeben des fusionierten Merkmals in ein mehrschichtiges Perzeptron, MLP, in der Ausgangsschicht und Zuordnen des fusionierten Merkmals unter Verwendung des MLP, um die Vorhersagewahrscheinlichkeit zu erhalten.

9. Verfahren nach Anspruch 1, wobei das Bestimmen einer Bindungsstelle der mindestens einen Stelle im Zielmolekül auf Basis der Vorhersagewahrscheinlichkeit jeder Stelle der mindestens einen Stelle Folgendes umfasst:
Bestimmen einer Stelle mit einer Vorhersagewahrscheinlichkeit, die größer ist als ein Wahrscheinlichkeitsschwellwert, von der mindestens einen Stelle als die Bindungsstelle.

10. Einrichtung zum Detektieren einer Molekülbindungsstelle, die Folgendes umfasst:

ein Erhaltungsmodul (701), das dazu ausgelegt ist, dreidimensionale 3D-Koordinaten mindestens einer Stelle in einem Zielmolekül zu erhalten, wobei das Zielmolekül ein chemisches Molekül mit einer zu detektierenden Bindungsstelle ist;
für jede Stelle der mindestens einen Stelle:

ein erstes Bestimmungsmodul (702), das dazu ausgelegt ist, einen ersten Zielpunkt und einen zweiten Zielpunkt zu bestimmen, wobei der erste Zielpunkt einen Mittelpunkt von allen Stellen in einem Kugelraum ist, wobei der Kugelraum ein Kugelraum mit der jeweiligen Stelle als einer Mitte einer Kugel und einer Ziellänge als einem Radius ist, und wobei der zweite Zielpunkt eine Überschneidung zwischen einer Vorwärtserstreckungslinie eines Vektors, beginnend bei einem Ursprung und auf die jeweilige Stelle zeigend, und einer Außenfläche des Kugelraums ist, wobei der Ursprung ein Ursprung eines 3D-Koordinatensystems ist, in dem sich das Zielmolekül befindet, und
ein Extraktionsmodul (703), das dazu ausgelegt ist, ein rotationsinvariantes Ortsmerkmal in den 3D-Koordinaten der jeweiligen Stelle auf Basis der 3D-Koordinaten der jeweiligen Stelle, 3D-Koordinaten des ersten Zielpunkts und 3D-Koordinaten des zweiten Zielpunkts zu extrahieren, wobei das rotationsinvariante Ortsmerkmal zum Anzeigen von Ortsinformationen der jeweiligen Stelle im Zielmolekül verwendet wird;
ein Vorhersagemodul (704), das dazu ausgelegt ist, ein Stellendetektionsmodell aufzurufen, um eine Vorhersage am extrahierten rotationsinvarianten Ortsmerkmal durchzuführen, um eine Vorhersagewahrscheinlichkeit jeder Stelle der mindestens einen Stelle zu erhalten, wobei jede Vorhersagewahrscheinlichkeit eine Wahrscheinlichkeit anzeigt, dass jede Stelle der mindestens einen Stelle eine Bindungsstelle ist; und
ein zweites Bestimmungsmodul (705), das dazu ausgelegt ist, eine Bindungsstelle der mindestens einen Stelle im Zielmolekül auf Basis der Vorhersagewahrscheinlichkeit jeder Stelle der mindestens einen Stelle zubestimmen.

11. Elektronische Vorrichtung, die einen oder mehrere Prozessoren und einen oder mehrere Speicher umfasst, wobei in dem einen oder den mehreren Speichern mindestens ein Teil eines Programmcodes gespeichert ist, wobei der mindestens eine Teil des Programmcodes von dem einen oder den mehreren Prozessoren geladen und ausgeführt wird, um das Verfahren zum Detektieren einer Molekülbindungsstelle gemäß einem der Ansprüche 1 bis 9 zu implementieren.

12. Speichermedium, auf dem mindestens ein Teil eines Programmcodes gespeichert ist, wobei der mindestens eine Teil des Programmcodes von einem Prozessor geladen und ausgeführt wird, um das Verfahren zum Detektieren einer Molekülbindungsstelle gemäß einem der Ansprüche 1 bis 9 zu implementieren.

**Revendications**

1. Procédé mis en œuvre par ordinateur pour détecter un site de liaison moléculaire, applicable à un dispositif électronique, le procédé comprenant les étapes suivantes :

obtenir (201) des coordonnées tridimensionnelles 3D d'au moins un site dans une molécule cible, la molécule cible étant une molécule chimique avec un site de liaison à détecter ;
pour chaque site du ou des sites :

déterminer (202) un premier point cible et un deuxième point cible, le premier point cible étant un point central de tous les sites dans un espace sphérique, l'espace sphérique étant un espace sphérique ayant le site respectif comme centre d'une sphère et une longueur cible comme rayon, et le deuxième point cible étant une intersection entre une ligne d'extension vers l'avant d'un vecteur partant d'une origine et pointant vers le site respectif et une surface extérieure de l'espace sphérique, l'origine étant une origine d'un système de coordonnées 3D dans lequel se situe la molécule cible, et

extraire (203) une caractéristique d'emplacement invariante en rotation dans les coordonnées 3D du site respectif sur la base des coordonnées 3D du site respectif, des coordonnées 3D du premier point cible et des coordonnées 3D du deuxième point cible, la caractéristique d'emplacement invariante en rotation étant utilisée pour indiquer les informations du site respectif dans la molécule cible ;

invoquer (204) un modèle de détection de site pour réaliser un traitement de prédiction sur la caractéristique d'emplacement invariante en rotation extraite afin d'obtenir une probabilité de prédiction de chacun des sites du ou des sites, chaque probabilité de prédiction indiquant une possibilité que chacun des sites du ou des sites soit un site de liaison ;

déterminer (205) un site de liaison à partir de l'au moins un site dans la molécule cible sur la base de la probabilité de prédiction de chacun des sites du ou des sites ;

dans lequel l'extraction de la caractéristique d'emplacement invariante en rotation dans les coordonnées 3D du site respectif comprend ce qui suit :

construire (302) une caractéristique d'emplacement global du site respectif sur la base des coordonnées 3D du site respectif, des coordonnées 3D du premier point cible et des coordonnées 3D du deuxième point cible, la caractéristique d'emplacement global étant utilisée pour indiquer des informations d'emplacement spatial du site dans la molécule cible ;

sur la base des coordonnées 3D du site respectif, des coordonnées 3D du premier point cible, des coordonnées 3D du deuxième point cible et de coordonnées 3D d'au moins un point de voisinage du site, construire (303) au moins une caractéristique d'emplacement local entre le site et l'au moins un point de voisinage, l'au moins une caractéristique d'emplacement local étant utilisée pour indiquer des informations d'emplacement relatif entre le site respectif et l'au moins un point de voisinage ; et

obtenir (304) la caractéristique d'emplacement du site respectif sur la base de la caractéristique d'emplacement global et de l'au moins une caractéristique d'emplacement local.

2. Procédé selon la revendication 1, dans lequel la caractéristique d'emplacement global comprend au moins une parmi une amplitude du site respectif, une distance entre le site respectif et le premier point cible, une distance entre le premier point cible et le deuxième point cible, une valeur cosinus d'un premier angle ou une valeur cosinus d'un deuxième angle, le premier angle étant un angle formé entre un premier segment de ligne et un deuxième segment de ligne, le deuxième angle étant un angle formé entre le deuxième segment de ligne et un troisième segment de ligne, le premier segment de ligne étant un segment de ligne formé entre le site respectif et le premier point cible, le deuxième segment de ligne étant un segment de ligne formé entre le premier point cible et le deuxième point cible, et le troisième segment de ligne étant un segment de ligne formé entre le site respectif et le deuxième point cible.

3. Procédé selon la revendication 1, dans lequel, pour tout point de voisinage du ou des points de voisinage, la caractéristique d'emplacement local entre le site respectif et le point de voisinage comprend au moins une parmi une distance entre le point de voisinage et le site, une distance entre le point de voisinage et le premier point cible, une distance entre le point de voisinage et le deuxième point cible, une valeur cosinus d'un troisième angle, une valeur cosinus d'un quatrième angle ou une valeur cosinus d'un cinquième angle, le troisième angle étant un angle formé entre un quatrième segment de ligne et un cinquième segment de ligne, le quatrième angle étant un angle formé entre le cinquième segment de ligne et un sixième segment de ligne, le cinquième angle étant un angle formé entre le sixième segment de ligne et le quatrième segment de ligne, le quatrième segment de ligne étant un segment de ligne formé entre le point de voisinage et le site respectif, le cinquième segment de ligne étant un segment de ligne formé entre le point de voisinage et le premier point cible, et le sixième segment de ligne étant un segment de ligne formé entre le point de voisinage et le deuxième point cible.

4. Procédé selon la revendication 1, dans lequel le modèle de détection de site est un réseau convolutif graphique, GCN, et le GCN comprend une couche d'entrée, au moins une couche convolutive de bord et une couche de sortie ; et l'invocation du modèle de détection de site pour réaliser un traitement de prédiction sur la caractéristique d'emplacement invariante en rotation extraite afin d'obtenir la probabilité de prédiction de chacun des sites du ou des sites comprend ce qui suit :

entrer (306) la caractéristique d'emplacement de chacun des sites du ou des sites dans la couche d'entrée du GCN, et délivrer des données graphiques de chacun des sites du ou des sites en utilisant la couche d'entrée, les données graphiques étant utilisées pour indiquer la caractéristique d'emplacement de chacun des sites du ou des sites sous la forme d'un graphe ;

entrer (307) les données graphiques de chacun des sites du ou des sites dans l'au moins une couche convolutive de bord du GCN, et réaliser une extraction de caractéristiques sur les données graphiques de chacun des sites du ou des sites en utilisant l'au moins une couche convolutive de bord afin d'obtenir une caractéristique biologique globale de chacun des sites du ou des sites ;

fusionner (308) la caractéristique biologique globale, les données graphiques de chacun des sites du ou des sites et une caractéristique convolutive de bord délivrée par l'au moins une couche convolutive de bord afin d'obtenir une caractéristique fusionnée ;

entrer (308) la caractéristique fusionnée dans la couche de sortie du GCN et en utilisant la couche de sortie, réaliser un ajustement de probabilité sur la caractéristique fusionnée afin d'obtenir la probabilité de prédiction.

5. Procédé selon la revendication 4, dans lequel l'entrée de la caractéristique d'emplacement de chacun des sites du ou des sites dans la couche d'entrée du GCN et la sortie des données graphiques de chacun des sites du ou des sites en utilisant la couche d'entrée comprennent ce qui suit :

entrer la caractéristique d'emplacement de chacun des sites du ou des sites dans une perceptron multicouche, MLP, de la couche d'entrée, et mapper la caractéristique d'emplacement de chacun des sites du ou des sites en utilisant la MLP afin d'obtenir une première caractéristique de chacun des sites du ou des sites, une quantité dimensionnelle de la première caractéristique étant supérieure à une quantité dimensionnelle de la caractéristique d'emplacement ; et

entrer la première caractéristique de chacun des sites du ou des sites dans une couche de mise en grappe de la couche d'entrée, et réaliser une réduction de dimension sur la première caractéristique de chacun des sites du ou des sites en utilisant la couche de mise en grappe afin d'obtenir les données graphiques de chacun des sites du ou des sites.

6. Procédé selon la revendication 4, dans lequel la réalisation de l'extraction de caractéristiques sur les données graphiques de chacun des sites du ou des sites en utilisant l'au moins une couche convolutive de bord afin d'obtenir une caractéristique biologique globale de chacun des sites du ou des sites comprend ce qui suit :

pour chaque couche convolutive de bord de la ou des couches convolutives de bord, réaliser une extraction de caractéristiques sur une caractéristique convolutive de bord délivrée par une couche convolutive de bord précédente, et entrer la caractéristique convolutive de bord extraite dans une couche convolutive de bord suivante ;

concaténer les données graphiques de chacun des sites du ou des sites et au moins une caractéristique convolutive de bord délivrée par l'au moins une couche convolutive de bord afin d'obtenir une deuxième caractéristique ;

entrer la deuxième caractéristique dans une perceptron multicouche, MLP, et mapper la deuxième caractéristique en utilisant la MLP afin d'obtenir une troisième caractéristique ; et

entrer la troisième caractéristique dans une couche de mise en grappe, et réaliser une réduction de dimension sur la troisième caractéristique en utilisant la couche de mise en grappe afin d'obtenir la caractéristique biologique globale.

7. Procédé selon la revendication 6, dans lequel la réalisation de l'extraction de caractéristiques sur une caractéristique convolutive de bord délivrée par une couche convolutive de bord précédente pour chaque couche convolutive de bord de la ou des couches convolutives de bord, et l'entrée de la caractéristique convolutive de bord extraite dans la couche convolutive de bord suivante comprennent ce qui suit :

construire une carte de grappe pour chaque couche convolutive de bord de la ou des couches convolutives de bord sur la base de la caractéristique convolutive de bord délivrée par la couche convolutive de bord précédente ;

entrer la carte de grappe dans une MLP de la couche convolutive de bord, et mapper la carte de grappe en utilisant la MLP afin d'obtenir une caractéristique intermédiaire de la carte de grappe ; et

entrer la caractéristique intermédiaire dans une couche de mise en grappe dans la couche convolutive de bord, réaliser une réduction de dimension sur la caractéristique intermédiaire en utilisant la couche de mise en grappe, et entrer la caractéristique intermédiaire à dimension réduite dans la couche convolutive de bord suivante.

**8.** Procédé selon la revendication 4, dans lequel l'entrée de la caractéristique fusionnée dans la couche de sortie du GCN, et la réalisation d'un ajustement de probabilité sur la caractéristique fusionnée en utilisant la couche de sortie afin d'obtenir la probabilité de prédiction comprennent ce qui suit :

entrer la caractéristique fusionnée dans une perceptron multicouche, MLP, dans la couche de sortie, et mapper la caractéristique fusionnée en utilisant la MLP afin d'obtenir la probabilité de prédiction.

**9.** Procédé selon la revendication 1, dans lequel la détermination du site de liaison à partir de l'au moins un site dans la molécule cible sur la base de la probabilité de prédiction de chacun des sites du ou des sites comprend ce qui suit :

déterminer un site du ou des sites ayant une probabilité de prédiction supérieure à un seuil de probabilité en tant que site de liaison.

**10.** Appareil pour détecter un site de liaison moléculaire, comprenant :

un module d'obtention (701) configuré pour obtenir des coordonnées tridimensionnelles 3D d'au moins un site dans une molécule cible, la molécule cible étant une molécule chimique avec un site de liaison à détecter ;
pour chaque site du ou des sites :

un premier module de détermination (702) configuré pour déterminer un premier point cible et un deuxième point cible, le premier point cible étant un point central de tous les sites dans un espace sphérique, l'espace sphérique étant un espace sphérique ayant le site respectif comme centre d'une sphère et une longueur cible comme rayon, et le deuxième point cible étant une intersection entre une ligne d'extension vers l'avant d'un vecteur partant d'une origine et pointant vers le site respectif et une surface extérieure de l'espace sphérique, l'origine étant une origine d'un système de coordonnées 3D dans lequel se situe la molécule cible, et
un module d'extraction (703) configuré pour extraire une caractéristique d'emplacement invariante en rotation dans les coordonnées 3D du site respectif sur la base des coordonnées 3D du site respectif, des coordonnées 3D du premier point cible et des coordonnées 3D du deuxième point cible, la caractéristique d'emplacement invariante en rotation étant utilisée pour indiquer les informations du site respectif dans la molécule cible ;
un module de prédiction (704) configuré pour invoquer un modèle de détection de site pour réaliser une prédiction sur la caractéristique d'emplacement invariante en rotation extraite afin d'obtenir une probabilité de prédiction de chacun des sites du ou des sites, chaque probabilité de prédiction indiquant une possibilité que chacun des sites du ou des sites soit un site de liaison ; et
un deuxième module de détermination (705) configuré pour déterminer un site de liaison à partir de l'au moins un site dans la molécule cible sur la base de la probabilité de prédiction de chacun des sites du ou des sites.

**11.** Dispositif électronique comprenant un ou plusieurs processeurs et une ou plusieurs mémoires, les une ou plusieurs mémoires stockant au moins un élément de code de programme, l'au moins un élément de code de programme étant chargé et exécuté par les un ou plusieurs processeurs pour mettre en œuvre le procédé pour détecter un site de liaison moléculaire selon l'une des revendications 1 à 9.

**12.** Support de stockage stockant au moins un élément de code de programme, l'au moins un élément de code de programme étant chargé et exécuté par un processeur pour mettre en œuvre le procédé pour détecter un site de liaison moléculaire selon l'une des revendications 1 à 9.

FIG. 1

The electronic device obtains 3D coordinates of at least one site in a target molecule, the target molecule including a chemical molecule with a to-be-detected binding site  `201`

The electronic device determines a first target point and a second target point, the first target point being a center point of all sites within a spherical space, the spherical space being a spherical space with the each of the at least one site as a center of a sphere and a target length as a radius, and the second target point being an intersection between a forward extension line of a vector, starting from an origin and pointing to the each of the at least one site, and an outer surface of the spherical space  `202`

The electronic device extracts a rotation-invariant location feature in the 3D coordinates of the each of the at least one site based on the 3D coordinates of the each of the at least one site, 3D coordinates of the first target point, and 3D coordinates of the second target point, the rotation-invariant location feature being used for indicating location information of the each of the at least one site in the target molecule  `203`

The electronic device invokes a site detection model to perform prediction processing on the extracted rotation-invariant location feature, to obtain a prediction probability of the each of the at least one site, the each prediction probability indicating a possibility of the each of the at least one site being a binding site  `204`

The electronic device determines a binding site in the at least one site in the target molecule based on the prediction probability of the each of the at least one site  `205`

FIG. 2

A terminal obtains 3D coordinates of at least one site in a target molecule to be detected, the target molecule being a chemical molecule with a binding site to be detected — 300

The terminal determines, for any site in the at least one site, a first target point and a second target point corresponding to the site based on the 3D coordinates of the site — 301

The terminal constructs a global location feature of the site based on the 3D coordinates of the site, 3D coordinates of the first target point, and 3D coordinates of the second target point, the global location feature being used for indicating spatial location information of the site in the target molecule — 302

The terminal constructs, based on the 3D coordinates of the site, the 3D coordinates of the first target point, the 3D coordinates of the second target point, and 3D coordinates of at least one neighborhood point of the site, at least one local location feature between the site and the at least one neighborhood point, one local location feature being used for indicating relative location information between the site and one neighborhood point — 303

The terminal obtains a location feature of the site based on the global location feature and the at least one local location feature — 304

The terminal repeats the foregoing steps 301 to 304 for the at least one site in the target molecule to obtain a location feature of the at least one site — 305

The terminal inputs the location feature of the at least one site into an input layer in a GCN, and outputs graph data of the at least one site by using the input layer, the graph data being used for indicating the location feature of the site in the form of a graph — 306

The terminal inputs the graph data of the at least one site into the at least one edge convolutional layer in the GCN, and performs feature extraction on the graph data of the at least one site by using the at least one edge convolutional layer, to obtain a global biological feature of the at least one site — 307

The terminal fuses the global biological feature, the graph data of the at least one site, and an edge convolutional feature outputted by the at least one edge convolutional layer, inputs a fused feature into an output layer of the GCN, and performs, by using the output layer, probability fitting on the fused feature, to obtain at least one prediction probability — 308

The terminal determines a binding site in from the at least one site in the target molecule based on the at least one prediction probability — 309

FIG. 3

402 403
dsm_i
$\beta_i$
$s_i$
$m_i$ $\alpha_i$
$dpm_i$
$p_i$ 400
$dp_i$
&
origin (0, 0, 0)
401

Protein molecule

Point cloud data

origin (0, 0, 0)
$r$
$p_i$

402 403
$\gamma_{ij}^p$ $p_{ij}$ $dps_{ij}$ $s_i$
$dpm_{ij}$ $\gamma_{ij}^s$
$m_i$ $\gamma_{ij}^m$ $dpp_{ij}$
$p_i$ 400

## FIG. 4

11
N
K
501

3
N
500

1
N
Detection result
508

MLPs

MLPs

32
N
K
502

Max pooling

32
N
503

Edge convolutional layer

64
N
504

Edge convolutional layer

128
N
505

MLPs
Max pooling

1024
506

1248
N
507

## FIG. 5

### Edge convolutional layer

C
N
601

KNN map
MLPs

C'
N
K
602

Pooling

C'
N
603

## FIG. 6

701

Obtaining module

702

First determining module

703

Extraction module

704

Prediction module

705

Second determining module

FIG. 7

800

803

801

Processor

802

Memory

Peripheral interface

804

Display screen

FIG. 8